# EUROPEAN PATENT APPLICATION

(11) **EP 4 261 232 A1**
(43) Date of publication of application: **18.10.2023**
(21) Application number: 21913268.5
(22) Date of filing: 26.09.2021
(51) Int. Cl.: C07K 19/00, C12N 15/62, C12N 5/10, A61P 35/00, A61K 39/12

(54) **HUMAN PAPILLOMAVIRUS TYPE 18 CHIMERIC PROTEIN AND USE THEREOF**

(30) Priority: 04.01.2021 CN 202110002251
(71) Applicant: Institute of Basic Medical Sciences, Chinese Academy of Medical Sciences, Beijing 100005 (CN)
(72) Inventor: XU, Xuemei, Beijing 100005 (CN); ZHANG, Ting, Beijing 100005 (CN); WANG, Zhirong, Beijing 100005 (CN); XIA, Baicheng, Beijing 100005 (CN)
(74) Representative: Danner, Stefan
(86) International application number: PCT/CN2021/120583
(87) International publication number: WO 2022/142523

(57) **Abstract**

The present invention relates to a human papillomavirus type 18 chimeric protein and the use thereof. Specifically, the present invention relates to a papillomavirus chimeric protein, which comprises or consists of an HPV type 18 L1 protein or a mutant of the HPV type 18 L1 protein, and a polypeptide from a HPV type 59 L2 protein inserted into the surface region of the HPV type 18 L1 protein or the mutant of the HPV type 18 L1 protein, wherein the amino acid sequence of the HPV type 18 L1 protein is as shown in SEQ ID No. 1, and the amino acid sequence of the HPV type 59 L2 protein is as shown in SEQ ID No. 2.

## Description

### FIELD OF THE INVENTION

The present invention relates to the field of biotechnology. Specifically, the present invention relates to a human papillomavirus chimeric protein, and a pentamer or a virus-like particle formed thereby, as well as use of the human papillomavirus chimeric protein, the human papillomavirus chimeric pentamer or the human papillomavirus chimeric virus-like particle in the preparation of a vaccine for the prevention of papillomavirus infections and infection-induced diseases.

### BACKGROUND OF THE INVENTION

Human papillomaviruses (HPVs) are a class of envelope-free small DNA viruses that infect epithelial tissues. More than 200 types of the virus have been identified and classified into α, β, γ, µ, and η genera according to the amino acid homology of L1, the main coat protein of human papillomavirus. They are also classified into mucosa types and skin types according to the different sites of infection. Mucosa type HPVs mainly infect the genitourinary, perianal and oropharyngeal mucosa and skin. They all belong to the α genus, and are further classified into oncogenic HPVs with transforming activity and low-risk HPVs (LR-HPVs) that induce benign hyperplasia. Oncogenic HPVs include 12 common high-risk types (including HPV16, -18, -31, -33, -35, -39, -45, -51, -52, -56, -58, -59, etc.), 1 probable high-risk type (HPV68), and more than 10 very rare possible high-risk types (HPV26, -30, -34, -53, -66, -67, -69, -70, -73, -82, -85, etc.). Studies have found that all oncogenic HPV-positive cancerous tissues show specific E6*I mRNA expression, decreased expression of tumor suppressor gene Rb/P53 and cyclin CD1, and increased expression of p16INK 4a, indicating that the oncogenic risk from infection with any type of oncogenic HPV is the same. There are about 12 types of low-risk HPVs (HPV6, -7, -11, -13, -32, -40, -42, -43, -44, -54, -74, -91, etc.), of which HPV6 and -11 types induce a total of 90% of perianal and genital condylomata acuminata and most of the recurrent respiratory papillomas. Skin-type HPVs mainly infect skin tissues other than the above sites, some of which (HPV2, -27, -57) induce skin verrucous hyperplasia, and other types (HPV5, -8, -38, etc.) are associated with the occurrence of squamous cell carcinoma and basal cell carcinoma of skin.

Malignant tumors that have been identified as related to oncogenic HPV infection are cervical cancer, vaginal cancer, vulval cancer, penile cancer, anal and perianal cancer, oropharyngeal cancer, tonsil cancer and oral cancer, among which cervical cancer is the most harmful. Cervical cancer is the third highest incidence of malignant tumor in women in the world, with an annual incidence of about 527,000, including 285,000 in Asia and 75,000 in China. The 12 common high-risk types of HPV induce a total of 95.2%-96.5% of cervical cancer, and the rest more than 10 probable and possible high-risk types induce a total of about 3.29% of cervical cancer. HPV type 16 is a prevalent high-risk type worldwide, with the highest detection rate in HPV-related tumors such as cervical cancer, perianal cancer, penile cancer, vulval cancer, and in precancerous lesions. HPV16 and HPV 18 have a detection rate of 50-60% and -20% in cervical cancer worldwide, respectively. The 12 common high-risk types of HPV induce a total of 95.2%-96.5% of cervical cancer, and the rest more than 10 probable and possible high-risk types induce a total of about 3.29% of cervical cancer.

HPV L1 virus-like particles (L1 VLPs) mainly induce type-specific neutralizing antibodies and protective responses, and the protective range of the vaccine can only be expanded by increasing the types of L1 VLPs. The three HPV vaccines on the market are all L1 VLP vaccines, namely the divalent vaccine by GSK (Cervarix, HPV16/-18), the tetravalent vaccine (Gardasil, HPV6/-11/-16/-18) and the nine-valent vaccine (Gardasil-9, HPV6/-11/-16/-18/-31/-33/-45/-52/-58) by Merck, of which the nine-valent vaccine with the widest protection range only covers 7 high-risk types and 2 low-risk types (HPV6/-11), and cannot prevent the skin types. In addition, the protection range of L1 VLP vaccines cannot be expanded by unlimitedly increasing the types of L1 VLPs. Therefore, L1 VLP vaccines can hardly meet the requirements of prevention of HPV infection-related diseases.

The secondary capsid protein L2 of HPV has no immune activity in its native state, but the N-terminal polypeptide of L2 can induce cross-neutralizing antibodies and cross-protection responses, although the immunogenicity is weak, the titer of the induced antibody is low, and the types cross-neutralized by monotypic L2 antiserum is limited. At present, a variety of conserved epitope peptides that can induce neutralizing antibodies have only been found in 16 L2N, in which aa. 17-38 is its main neutralizing epitope region, and the monoclonal antibody RG-1 recognizing this region cross-neutralizes the most types. Therefore, this region is also called RG-1 epitope peptide, in which aa. 21-31 is the core sequence of its neutralizing epitope. The homologous region of aa. 21-31 is always retained in studies related to RG-1 epitope peptides, regardless of the length of the sequence.

The RG-1 types that have been reported for vaccine research include HPV type 4 RG-1, HPV type 6 RG-1, HPV type 16 RG-1, HPV type 17 RG-1, HPV type 31 RG-1, HPV type 33 RG-1, HPV type 45 RG-1, HPV type 51 RG-1, HPV type 58 RG-1, etc. [C. Schellenbacher et al., The Journal of investigative dermatology 2013, 133(12):2706-13; H. Seitz et al., Vaccine 2014, 32(22):2610-2617; B. Huber et al., PLoS One 2015, 10(3): e0120152; B. Huber et al., PLoS One 2017, 12(1):e0169533; X. Chen et al., Oncotarget 2017, 8(38):63333-63344; X. Chen et al., Human Vaccines & Immuno- therapeutics 2018, 14(8):2025-2033; PCT/CN2017/075402], and the employed means include VLP surface display, bacterial protein surface display (bacterial thioredededin Trx, flagellar protein, cholera toxin mutant CRM197), targeted IgyR modified antibody and tandem fusion of multiple types of L2 polypeptides containing the RG-1 epitope. However, studies have shown that a variety of RG-1 epitope peptide-related vaccines have poor activity results. For example, the three type 16 cVLPs surface displaying HPV type 4 RG-1, HPV type 6 RG-1 or HPV type 17 RG-1 induced very low titer of HPV16 neutralizing antibody, and the titer of cross-neutralization was not detected [B. Huber et al., PLoS One 2017, 12(1):e0169533; X. Chen et al., Oncotarget 2017, 8(38):63333-63344]. The 18cVLP displaying HPV type 45 RG-1 induced very low titer of HPV18 neutralizing antibody (only 1/100 of type 18 L1VLP), and only cross-neutralized the oncogenic types HPV45, -70 and -39 with very low titer, the highest was only 100 [B. Huber et al., PLoS One 2015, 10(3):e0120152]. The Trx fusion protein antiserum surface displaying type 51 RG1 had a narrow cross-neutralization range, and the highest titer of cross-neutralizing antibody was only 500 [H. Seitz et al., Vaccine 2014, 32(22):2610-2617]. In contrast, the type 16 RG1-cVLP reported by Schellenbacher et al. as well as the type 31 RG1-cVLP, type 33 RG1-cVLP and type 58 RG1-cVLP previously reported by the inventors had better immunoactivity, and the titer of HPV16 neutralizing antibody induced by the backbone type VLP was as high as 105 (comparable to that induced by type 16 L1 VLP), and the corresponding RG-1 epitope-induced L2-dependent cross-neutralizing antibodies had a wide neutralization range and relatively high titers (the highest was up to 6400) [C. Schellenbacher et al., The Journal of investigative dermatology 2013, 133(12):2706-13; X. Chen et al., Oncotarget 2017, 8(38):63333-63344; X. Chen et al., Human Vaccines & Immunotherapeutics 2018, 14(8):2025-2033; PCT/CN2017/075402].

The above data suggest that the RG-1 epitope peptides derived from different HPV types have very different immunogenicity. The inventors compared the immunogenicity of type 58 RG-1 and type 6 RG-1 in a previous publication, and found that type 58 RG-1 epitope peptide antiserum cross-neutralized more types (13 types) and had higher titers (the highest was up to 3200), while type 6 RG-1 epitope peptide antiserum neutralized fewer types (9 types) and had very low titers (the highest was only 100) [X. Chen et al., Oncotarget 2017, 8(38):63333-63344]. This indicated that although the RG-1 epitope peptide region was strongly conserved among different types, the different types of RG-1 had different immunogenicity. Therefore, the immune activity of a chimeric protein vaccine constructed with 17-36 homologous polypeptide selected from any type of L2 cannot be expected.

In another aspect, the HPV16 cVLP vaccine study reported by Schellenbacher and Wang showed that when inserting a type 16 RG-1 epitope peptide into the surface region of a type 16 L1 VLP vector, due to the difference in the flanking sequence, the insertion site and insertion method of the type 16 RG-1 core epitope peptide sequence, the immune activity of the multiple different type 16 RG1-cVLPs obtained was significantly different. Among them, the cVLP with insertion of type 16 RG-1 into the DE loop region of type 16 L1 had the best activity, and the cVLP with insertion of type 16 RG-1 core sequence into the h4 region of type 16 L1 had the poorest activity. In addition, Chen and Boxus both reported the cVLP of type 33 RG-1, but different vectors, HPV16 L1 VLP and 18 L1 VLP were used respectively. Although both reports chose the DE loop as the insertion site, but the insertion region differed by 1 amino acid, and the epitope peptide length differed by 2 amino acids. The two types 33 RG1-cVLPs induced very significantly different activity of the type 33 RG-1-dependent cross-neutralizing antibodies. Type 33 RG1-cVLP antiserum could cross-neutralize at least 12 types (2 types of which had a titer > 1000), while type 33 RG1-18cVLP antiserum only cross-neutralized 7 types, of which 6 types had a much lower neutralization titer (4 types of which had a titer < 100) than the 33RG1-16cVLP antiserum.

Therefore, there is a current need to develop a vaccine based on HPV L1 and HPV L2 chimeric proteins that can produce high-titer neutralizing antibodies against more types of HPV viruses.

### SUMMARY OF THE INVENTION

In order to solve the above technical problems, the inventors selected a variety of different lengths of type 59 RG-1 epitope peptides for research on HPV cVLPs. The results showed that the HPV type 18 cVLP obtained in the present invention had very strong immunogenicity, and induced serum neutralizing antibodies that could neutralize multiple types of HPV of the α7 subgenus at high titer.

The inventors performed comparative analysis of the neutralization activity of 8 different types of RG-1 epitope immune serum in Example 1. The inventors unexpectedly found that the immune serum of type 59 RG-1 epitope peptide could cross-neutralize at least 17 types, in particular, the titers of neutralizing HPV type 45, 59 and 16 were all above 103. It had the best activity of neutralizing the high-risk HPV types of the α7 subgenus reported so far, and the cross-neutralization activity against HPV16 was comparable to 16RG-1, currently reported as having a relatively stronger immunogenicity.

In view of this, the object of the present invention is to provide a human papillomavirus chimeric protein for the preparation of a vaccine for the prevention of papillomavirus infection and infection-induced diseases.

The present invention is based on the following unexpected findings of the inventors: the insertion of HPV type 59 L2 protein polypeptide into the surface region of the full-length or truncated HPV type 18 L1 protein can improve the immunogenicity of HPV type 59 L2 protein polypeptide. The obtained chimeric protein can be expressed at a high level in an *E. coli* or insect cell expression system. The chimeric protein can be assembled into VLP, and can induce a broad-spectrum protective immune response against multiple types of HPVs from different genera/subgenera. Relevant experimental results are provided in the examples herein.

Based on the above object, in one aspect, the present invention provides a human papillomavirus chimeric protein, the backbone of which is a HPV18 L1 protein or a mutant of the HPV18 L1 protein, and the backbone is chimeric with at least one polypeptide from a HPV type 59 L2 protein.

That is, in a first aspect of the present invention, the present invention provides a human papillomavirus chimeric protein comprising or consisting of a HPV type 18 L1 protein or a mutant of the HPV type 18 L1 protein, and a polypeptide from a HPV type 59 L2 protein inserted into the surface region of the HPV type 18 L1 protein or the mutant of the HPV type 18 L1 protein, wherein the amino acid sequence of the HPV type 18 L1 protein is as shown in SEQ ID No. 1, and the amino acid sequence of the HPV type 59 L2 protein is as shown in SEQ ID No. 2.

In a preferred embodiment of the human papillomavirus chimeric protein according to the present invention, the mutant of the HPV type 18 L1 protein is a protein obtained by truncating 0-8 amino acids at N-terminus and/or truncating 0-32 amino acids at C-terminus of the HPV type 18 L1 protein.

In a preferred embodiment of the human papillomavirus chimeric protein according to the present invention, the mutant of the HPV type 18 L1 protein is selected from the group consisting of:
a mutant with a 32-amino acid truncation at C-terminus of the amino acid sequence as shown in SEQ ID No. 1;
a mutant with amino acid substitutions of amino acids 477, 478, 484, 496, 499, 504, 506 to glycine (G) and amino acids 485, 500, 502 to serine (S) in the amino acid sequence as shown in SEQ ID No. 1 (mut1);
a mutant with amino acid substitutions of amino acids 477, 478, 485, 496, 499, 504, 506 to glycine (G) and amino acids 486, 500, 502 to serine (S) in the amino acid sequence as shown in SEQ ID No. 1 (mut2);
a mutant with amino acid substitutions of amino acids 477, 478, 484, 496, 499, 502, 506 to glycine (G), amino acids 485, 500 to serine (S) and amino acid 504 to aspartate (D) in the amino acid sequence as shown in SEQ ID No. 1 (mut3);
a mutant with amino acid substitutions of amino acids 477, 478, 485, 496, 502, 506 to glycine (G), amino acids 486, 500 to serine (S) and amino acids 499, 504 to aspartate (D) in the amino acid sequence as shown in SEQ ID No. 1 (mut4);
a mutant with amino acid substitutions of amino acids 477, 484, 496, 499, 504, 506 to glycine (G) and amino acids 485, 500, 502 to serine (S) in the amino acid sequence as shown in SEQ ID No. 1 (mut5); and
a mutant with amino acid substitutions of amino acids 477, 485, 496, 499, 504, 506 to glycine (G) and amino acids 486, 500, 502 to serine (S) in the amino acid sequence as shown in SEQ ID No. 1 (mut6).

In a preferred embodiment of the human papillomavirus chimeric protein according to the present invention, the polypeptide from the HPV type 59 L2 protein is selected from any continuous fragment of 8-33 amino acids in the region of amino acids 1-50 as shown in SEQ ID No. 2. Preferably, the polypeptide from the HPV type 59 L2 protein is a HPV type 59 L2 protein RG-1 epitope peptide or a mutant epitope peptide thereof. Further preferably, the polypeptide from HPV type 59 L2 protein consists of an amino acid sequence selected from the group consisting of amino acids 17 to 32 as shown in SEQ ID No. 2, amino acids 16 to 35 as shown in SEQ ID No. 2, amino acids 17 to 37 as shown in SEQ ID No. 2, amino acids 16 to 37 as shown in SEQ ID No. 2, and a sequence with extension or truncation of 1 to 7 amino acids at N-terminus and/or C-terminus of the above amino acid sequences.

Most preferably, the amino acid sequence of the polypeptide from HPV type 59 L2 protein is as shown in SEQ ID No. 3, SEQ ID No. 4, SEQ ID No. 5 or SEQ ID No. 6.

Optionally, the polypeptide from HPV type 59 L2 protein is a polypeptide obtained by extending or truncating 1-7 amino acids at N-terminus and/or extending or truncating 1-7 amino acids at C-terminus of the amino acid sequence shown in SEQ ID No. 3.

Optionally, the polypeptide from HPV type 59 L2 protein can further be a polypeptide with greater than 60%, preferably greater than 70%, preferably greater than 80%, greater than 90%, and even more preferably greater than 95% of sequence identity with the amino acid sequence as shown in SEQ ID No. 3, SEQ ID No. 4, SEQ ID No. 5 or SEQ ID No. 6.

In a preferred embodiment of the human papillomavirus chimeric protein according to the present invention, the HPV type 18 L1 protein can be from, for example but not limited to, the L1 proteins from HPV18 variant strains, such as ATL15214.1, ATL14646.1, ARS43458.1, ARS43428.1, ARS43449.1, AGU90430.1 in the NCBI database. Preferably, the amino acid sequence of the HPV type 18 L1 protein is as shown in SEQ ID No. 1.

In a preferred embodiment of the human papillomavirus chimeric protein according to the present invention, the polypeptide from the HPV type 59 L2 protein is inserted into the surface region of the HPV type 18 L1 protein or the mutant of the HPV type 18 L1 protein, preferably inserted into the DE loop and/or h4 region of the HPV type 18 L1 protein or the mutant of the HPV type 18 L1 protein. More preferably, the polypeptide from the HPV type 59 L2 protein is inserted between amino acids 134 and 135, or between amino acids 137 and 138, or between amino acids 432 and 433, or between amino acids 434 and 435 of the HPV type 18 L1 protein or the mutant of the HPV type 18 L1 protein by direct insertion, alternatively inserted into the region of amino acids 121 to 124, or the region of amino acids 131 to 138, or the region of amino acids 431 to 433, or the region of amino acids 432 to 435 of the HPV type 18 L1 protein or the mutant of the HPV type 18 L1 protein by non-isometric substitution.

As used herein, the term "direct insertion" refers to the insertion of a selected peptide fragment between two adjacent amino acids. For example, direct insertion between amino acids 134 and 135 of SEQ ID No. 1 refers to the direct insertion of the selected peptide fragment between amino acids 134 and 135 of SEQ ID No. 1.

As used herein, the term "non-isometric substitution" refers to the insertion of a selected peptide fragment into the specified amino acid region after deleting the sequence of the specified amino acid region. For example, non-isometric substitution into the region of amino acids 121 to 124 of SEQ ID No. 1 refers to the insertion of the selected peptide fragment between amino acids 121 and 124 of SEQ ID No. 1 after deleting amino acids 122 to 123 of SEQ ID No. 1. Optionally, in the embodiments of direct insertion or non-isometric substitution, the polypeptide from the HPV type 59 L2 protein comprises a linker of 1 to 3 amino acid residues in length at its N-terminus and/or C-terminus.

Optionally, the linker consists of any combination of amino acids selected from the group consisting of glycine (G), serine (S), alanine (A) and proline (P). Preferably, a G (glycine) P (proline) linker is selected for the N-terminus, and a P (proline) linker is selected for the C-terminus.

In a preferred embodiment of the papillomavirus chimeric protein of the present invention, in the embodiment of direct insertion, the amino acid sequence of the polypeptide from the HPV type 59 L2 protein is SEQ ID No. 4 or SEQ ID No. 5, the insertion site is between amino acids 137 and 138 of the HPV type 18 L1 protein or the mutant of the HPV type 18 L1 protein with 32-amino acid truncation at C-terminus, and the amino acid sequence of the obtained papillomavirus chimeric protein is as shown in SEQ ID No. 7, SEQ ID No. 8, SEQ ID No. 9 or SEQ ID No. 10.

In a preferred embodiment of the human papillomavirus chimeric protein of the present invention, in the embodiment of direct insertion, the amino acid sequence of the polypeptide from the HPV type 59 L2 protein is SEQ ID No. 3, the insertion site is between amino acids 432 and 433 or between amino acids 434 and 435 of the HPV type 18 L1 protein or the mutant of the HPV type 18 L1 protein with 32-amino acid truncation at C-terminus, and the amino acid sequence of the obtained papillomavirus chimeric protein is as shown in SEQ ID No. 11, SEQ ID No. 12, SEQ ID No. 13 or SEQ ID No. 14.

In a preferred embodiment of the human papillomavirus chimeric protein of the present invention, in the embodiment of direct insertion, the amino acid sequence of the polypeptide from the HPV type 59 L2 protein is the sequence as shown in SEQ ID No. 4 or SEQ ID No. 6 containing a GP linker at N-terminus and a P linker at C-terminus, the insertion site is between amino acids 134 and 135 of the HPV type 18 L1 protein or the mutant of the HPV type 18 L1 protein with 32-amino acid truncation at C-terminus, and the amino acid sequence of the obtained papillomavirus chimeric protein is as shown in SEQ ID No. 15, SEQ ID No. 16, SEQ ID No. 17 or SEQ ID No. 18.

In a preferred embodiment of the human papillomavirus chimeric protein of the present invention, in the embodiment of non-isometric substitution, after deleting the region of amino acids 132-137 of the HPV type 18 L1 protein or the mutant of the HPV type 18 L1 protein with 32-amino acid truncation at C-terminus, a polypeptide from the HPV type 59 L2 protein is inserted between the amino acids 131 and 138 of the HPV type 18 L1 protein or the mutant of the HPV type 18 L1 protein with 32-aa truncation at C-terminus, the polypeptide from HPV type 59 L2 protein has a glycine-proline linker added at its N-terminus, the amino acid sequence of the polypeptide from HPV type 59 L2 protein is as shown in SEQ ID No. 3, and the amino acid sequence of the obtained papillomavirus chimeric protein is as shown in SEQ ID No. 19 or SEQ ID No. 20.

In a preferred embodiment of the human papillomavirus chimeric protein of the present invention, in the embodiment of non-isometric substitution, after deleting the region of amino acids 122-123 of the HPV type 18 L1 protein or the mutant of the HPV type 18 L1 protein with 32-aa truncation at C-terminus, a polypeptide from the HPV type 59 L2 protein is inserted between the amino acids 121 and 124 of the HPV type 18 L1 protein or the mutant of the HPV type 18 L1 protein with 32-amino acid truncation at C-terminus. In the embodiment of the direct insertion, the amino acid sequence of the polypeptide from the HPV type 59 L2 protein is as shown in SEQ ID No. 3, and the amino acid sequence of the obtained papillomavirus chimeric protein is as shown in SEQ ID No. 21 or SEQ ID No. 22.

In a preferred embodiment of the human papillomavirus chimeric protein of the present invention, in the embodiment of non-isometric substitution, after deleting the amino acid 432 of the HPV type 18 L1 protein or the mutant of the HPV type 18 L1 protein with 32-aa truncation at C-terminus, a polypeptide from the HPV type 59 L2 protein is inserted between the amino acids 431 and 433 of the HPV type 18 L1 protein or the mutant of the HPV type 18 L1 protein with 32-amino acid truncation at C-terminus. In the embodiment of the direct insertion, the amino acid sequence of the polypeptide from the HPV type 59 L2 protein is as shown in SEQ ID No. 3, and the amino acid sequence of the obtained papillomavirus chimeric protein is as shown in SEQ ID No. 23 or SEQ ID No. 24.

In a preferred embodiment of the human papillomavirus chimeric protein of the present invention, in the embodiment of non-isometric substitution, after deleting the amino acids 433-434 of the HPV type 18 L1 protein or the mutant of the HPV type 18 L1 protein with 32-aa truncation at C-terminus, a polypeptide from the HPV type 59 L2 protein is inserted between the amino acids 432 and 435 of the HPV type 18 L1 protein or the mutant of the HPV type 18 L1 protein with 32-amino acid truncation at C-terminus. In the embodiment of the direct insertion, the amino acid sequence of the polypeptide from the HPV type 59 L2 protein is as shown in SEQ ID No. 3, and the amino acid sequence of the obtained papillomavirus chimeric protein is as shown in SEQ ID No. 25 or SEQ ID No. 26.

In a preferred embodiment of the human papillomavirus chimeric protein of the present invention, the polypeptide as shown in SEQ ID No. 4 is inserted between amino acids 137 and 138 of the mutant of the HPV type 18 L1 protein by direct insertion, and the mutant of the HPV type 18 L1 protein is selected from the group consisting of:
a mutant with amino acid substitutions of amino acids 477, 478, 484, 496, 499, 504, 506 to glycine (G) and amino acids 485, 500, 502 to serine (S) in the amino acid sequence as shown in SEQ ID No. 1, and the amino acid sequence of the obtained papillomavirus chimeric protein is as shown in SEQ ID No. 27 (mut1); or,
a mutant with amino acid substitutions of amino acids 477, 478, 485, 496, 499, 504, 506 to glycine (G) and amino acids 486, 500, 502 to serine (S) in the amino acid sequence as shown in SEQ ID No. 1, and the amino acid sequence of the obtained papillomavirus chimeric protein is as shown in SEQ ID No. 28 (mut2); or,
a mutant with amino acid substitutions of amino acids 477, 478, 484, 496, 499, 502, 506 to glycine (G), amino acids 485, 500 to serine (S) and amino acid 504 to aspartate (D) in the amino acid sequence as shown in SEQ ID No. 1, and the amino acid sequence of the obtained papillomavirus chimeric protein is as shown in SEQ ID No. 29 (mut3); or,
a mutant with amino acid substitutions of amino acids 477, 478, 485, 496, 502, 506 to glycine (G) and amino acids 486, 500 to serine (S) and amino acids 499, 504 to aspartate (D) in the amino acid sequence as shown in SEQ ID No. 1, and the amino acid sequence of the obtained papillomavirus chimeric protein is as shown in SEQ ID No. 30 (mut4); or,
a mutant with amino acid substitutions of amino acids 477, 484, 496, 499, 504, 506 to glycine (G) and amino acids 485, 500, 502 to serine (S) in the amino acid sequence as shown in SEQ ID No. 1, and the amino acid sequence of the obtained papillomavirus chimeric protein is as shown in SEQ ID No. 31 (mut5); or,
a mutant with amino acid substitutions of amino acids 477, 485, 496, 499, 504, 506 to glycine (G) and amino acids 486, 500, 502 to serine (S) in the amino acid sequence as shown in SEQ ID No. 1, and the amino acid sequence of the obtained papillomavirus chimeric protein is as shown in SEQ ID No. 32 (mut6).

In another aspect, the present invention relates to a polynucleotide encoding the above papillomavirus chimeric protein.

The present invention also provides a vector comprising the above polynucleotide, as well as a cell comprising the vector.

The polynucleotide sequence encoding the above papillomavirus chimeric protein of the present invention is suitable for different expression systems. Optionally, these nucleotide sequences are whole-gene optimized with *E*. *coli* codons and can be expressed at high levels in an *E. coli* expression system; alternatively, they are whole-gene optimized with insect cell codons and can be expressed at high levels in an insect cell expression system.

The present invention also provides a polymer, preferably, the polymer is a papillomavirus chimeric pentamer or chimeric virus-like particle comprising or formed by the above papillomavirus chimeric protein.

The present invention also provides use of the above papillomavirus chimeric protein, the papillomavirus chimeric pentamer or the papillomavirus chimeric virus-like particle in the preparation of a vaccine for the prevention of papillomavirus infection and/or papillomavirus infection-induced diseases, preferably, the papillomavirus infection-induced diseases include but are not limited to cervical, vaginal, vulval, penile, perianal, oropharyngeal, tonsil and oral cancers;
preferably, the papillomavirus infection is an infection selected from one or more of the following papillomavirus types: HPV16, HPV18, HPV26, HPV31, HPV33, HPV35, HPV39, HPV45, HPV51, HPV52, HPV53, HPV56, HPV58, HPV59, HPV66, HPV68, HPV70, HPV73; HPV6, HPV11, HPV2, HPV5, HPV27 and HPV57.

The present invention also provides a vaccine for the prevention of papillomavirus infection and infection-induced diseases, comprising the above papillomavirus chimeric pentamer or chimeric virus-like particle, an adjuvant, and an excipient or carrier for vaccines, preferably further comprising at least one virus-like particle or chimeric virus-like particle of mucosa-tropic and/or skin-tropic HPVs. Wherein, the content of these virus-like particles is an effective amount that can separately induce a protective immune response. Optionally, the adjuvant is an adjuvant for human use.

### Description and explanation of relevant terms in the present invention

According to the present invention, the term "insect cell expression system" includes insect cell, recombinant baculovirus, recombinant Bacmid and expression vector. Among them, the insect cell is derived from commercially available cells, the examples of which are listed here but are not limited to: Sf9, Sf21, High Five.

According to the present invention, the term "prokaryotic expression system" includes but is not limited to *E*. *coli* expression system. Wherein, the expression host bacteria are derived from commercially available strains, the examples of which are but are not limited to: BL21 (DE3), BL21 (DE3) plysS, C43 (DE3), Rosetta-gami B (DE3).

According to the present invention, examples of the term "full-length HPV type 18 L1 protein" include, but are not limited to, full-length L1 protein with a length equal to the protein No. ATL15070.1 in the NCBI database.

The gene fragment of "truncated HPV type 18 L1 protein" means that it has deletion of nucleotides encoding 1 or more amino acids at its 5' end and/or 3' end compared to the gene of wild-type HPV type 18 L1 protein, wherein the full-length sequence of "wild-type HPV type 18 L1 protein" is for example, but is not limited to, the following sequences in the NCBI database: ATL15214.1, ATL14646.1, ARS43458.1, ARS43428.1, ARS43449.1, AGU90430.1, etc.

According to the present invention, the term "excipient or carrier for vaccines" refers to that selected from one or more of the following, including but not limited to pH adjuster, surfactant and ionic strength enhancer. For example, the pH adjuster is for example but not limited to phosphate buffer. The surfactant includes cationic, anionic or nonionic surfactant, and is for example but not limited to polysorbate 80 (Tween-80). The ionic strength enhancer is for example but not limited to sodium chloride.

According to the present invention, the term "adjuvant for human use" refers to an adjuvant that can be applied clinically to the human body, including various adjuvants that have been approved and may be approved in the future, for example, but not limited to, aluminum adjuvant, MF59 and various forms of adjuvant compositions.

According to the present invention, the vaccine of the present invention can be in a patient-acceptable form, including oral administration or injection, preferably injection.

According to the present invention, the vaccine of the present invention is preferably used in a unit dosage form, wherein the dose of protein virus-like particles in the unit dosage form is 5 µg to 100 µg, for example, 5, 10, 15, 20, 25, 30, 35, 40, 45, 50, 55, 60, 65, 70, 75, 80, 85, 90, 95, 100 µg, as well as the range between any two of the above values, preferably 30 µg to 60 µg.

### DESCRIPTION OF THE DRAWINGS

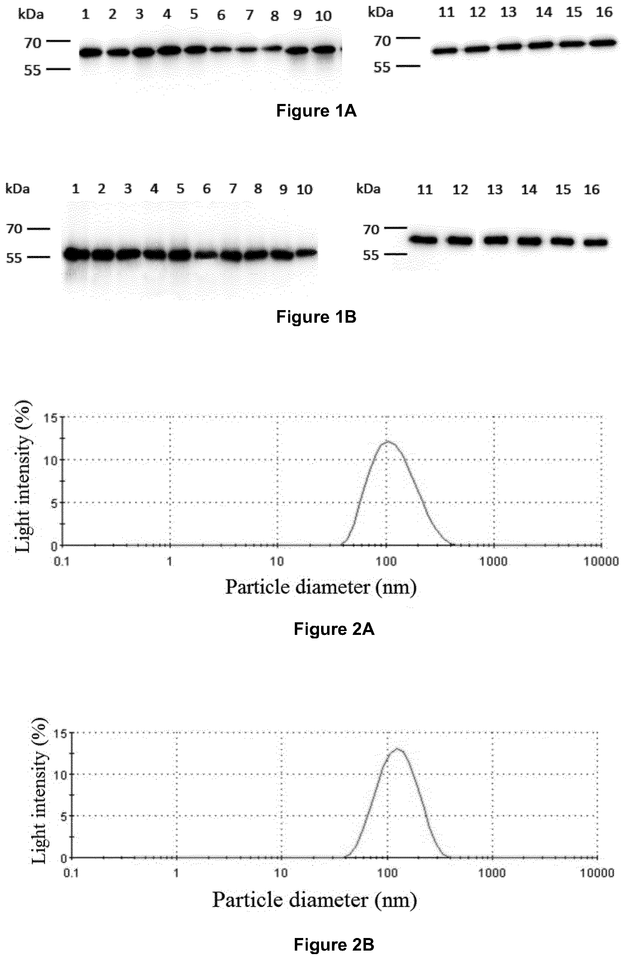

**Figure 1A to Figure 1B****:** Identification of the expression of chimeric proteins in Example 6 of the present invention in *E. coli* and insect cells. The results showed that all 26 chimeric proteins could be expressed in *E. coli* or insect cells, of which 6 chimeric proteins could be expressed in both expression systems. **Figure 1A****:** Identification of the expression of the chimeric proteins in *E. coli*: 1 represents 18L1DE137-138/59dES; 2 represents 18L1DE137-138/59dE; 3 represents 18L1h4432- 433/59dE; 4 represents 18L1h4434-435/59dE; 5 represents 18L1DE134-135/59dES; 6 represents 18L1DE134-135/59dE; 7 represents 18L1DE131-138/59dE; 8 represents 18L1DE121-124/59dE; 9 represents 18L1h4431-433/59dE; 10 represents 18L1h4432-435/59dE; 11 represents 18L1DE137-138/59dES-mut1; 12 represents 18L1DE137-138/59dES-mut2; 13 represents 18L1DE137-138/59dES-mut3; 14 represents 18L1DE137-138/59dES-mut4; 15 represents 18L1DE137- 138/59dES-mut5; 16 represents 18L1DE137-138/59dES-mut6; **Figure 1B****:** Identification of the expression of the chimeric proteins in insect cells: 1 represents 18L1ΔCDE137-138/59dES; 2 represents 18L1ΔCDE137-138/59dE; 3 represents 18L1ΔCh4432-433/59dE; 4 represents 18L1ΔCh4434-435/59dE; 5 represents 18L1ΔCDE134-135/59dES; 6 represents 18L1ΔCDE134- 135/59dE; 7 represents 18L1ΔCDE131-138/59dE; 8 represents 18L1ΔCDE121-124/59dE; 9 represents 18L1ΔCh4431-433/59dE; 10 represents 18L1ΔCh4432-435/59dE; 11 represents 18L1DE137-138/59dES-mut1; 12 represents 18L1DE137-138/59dES-mut2; 13 represents 18L1DE137-138/59dES-mut3; 14 represents 18L1DE137-138/59dES-mut4; 15 represents 18L1DE137-138/59dES-mut5; 16 represents 18L1DE137- 138/59dES-mut6.

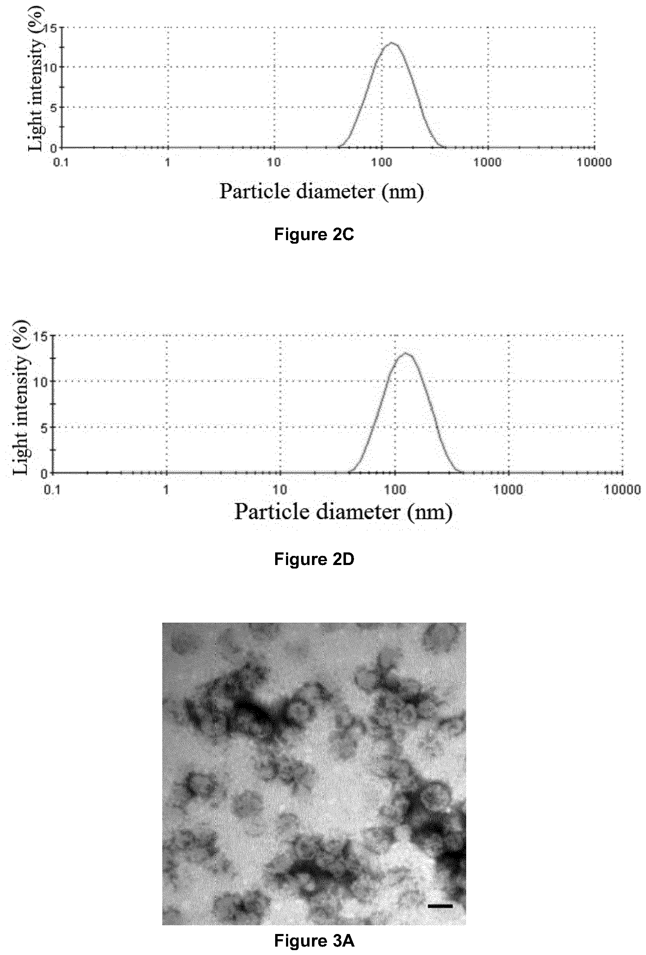

**Figure 2A to Figure 2D****:** Dynamic light scattering analysis results of cVLPs obtained after purification in Example 6 of the present invention. The results showed that the hydraulic diameters of virus-like particles formed by 18L1ΔCDE134-135/59dE, 18L1ΔCDE134-135/59dES, 18L1ΔCDE137-138/59dE and 18L1ΔCDE137-138/59dES recombinant proteins were 106.8 nm, 113.3 nm, 114.7 nm and 122.9 nm, respectively, and the percentages of particle assembly were all 100%. **Figure 2A****:** 18L1ΔCDE134-135/59dE; **Figure 2B****:** 18L1ΔCDE134-135/59dES; **Figure 2C****:** 18L1ΔCDE137-138/59dE; **Figure 2D****:** 18L1ΔCDE137-138/59dES.

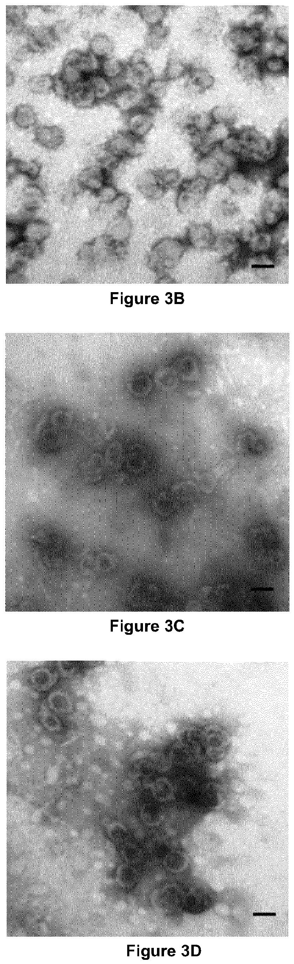

**Figure 3A to Figure 3D****:** Transmission electron microscopy observation results of cVLPs obtained after purification in Example 7 of the present invention. A large number of virus-like particles could be seen in the field, and the particles had good uniformity. The diameter of cVLPs was about 50 nm, similar to the size of VLPs of the L1 protein. Bar = 50 nm. **Figure 3A****:** 18L1ΔCDE134-135/59dE; **Figure 3B****:** 18L1ΔCDE134-135/59dES; **Figure 3C****:** 18L1ΔCDE137- 138/59dE; **Figure 3D****:** 18L1ΔCDE137-138/59dES.

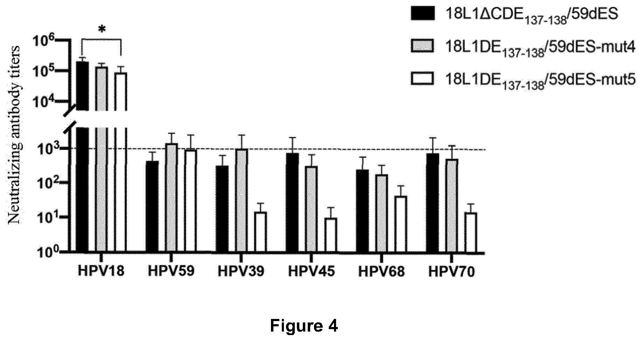

**Figure 4****:** Detection of the neutralizing activity of the chimeric VLP mouse immune serum in Example 10 of the present invention against HPV pseudovirus of the α7 subgenus. *: P<0.05.

### DETAILED DESCRIPTION OF THE INVENTION

The present invention will be further illustrated below by the following non-limiting examples. It is well known to those skilled in the art that many modifications can be made to the present invention without departing from the spirit of the present invention, and such modifications also fall within the scope of the present invention. The following embodiments are only used to illustrate the present invention and should not be regarded as limiting the scope of the present invention, as the embodiments are necessarily diverse. The terms used in the present specification are intended only to describe particular embodiments but not as limitations. The scope of the present invention has been defined in the appended claims.

Unless otherwise specified, all the technical and scientific terms used in the present specification have the same meaning as those generally understood by those skilled in the technical field to which the present application relates. Preferred methods and materials of the present invention are described below, but any method and material similar or equivalent to the methods and materials described in the present specification can be used to implement or test the present invention. Unless otherwise specified, the following experimental methods are conventional methods or methods described in product specifications. Unless otherwise specified, the experimental materials used are easily available from commercial companies. All published literatures referred to in the present specification are incorporated here by reference to reveal and illustrate the methods and/or materials in the published literatures.

### Example 1: Detection of immunoactivitv of different types of RG-1 epitope peptides

HPV35, -39, -51, -53, -56, -59, -68, -82 RG-1 epitope peptides were synthesized using chemical synthesis, and the sequences of the epitope peptides were as shown in Table 1. The polypeptides were synthesized by GL Biochem (Shanghai) Co., Ltd. In order to improve the immunegenicity of the synthetic peptides, each synthetic peptide was coupled with keyhole limpet hemocyanin (KLH) after activation of carboxyl group by 1-(3-dimethylaminopropyl)-3-ethyl- carbodiimide hydrochloride (EDC, CAS No. 25952-53-8).

New Zealand white rabbits weighing 2.0-2.5 kg were randomly divided into groups, 2-4 rabbits per group. Four days before immunization, 15 mg of inactivated DH5a (PBS containing 0.5% v/v formaldehyde, treated at 37°C for 24-48 h) thoroughly mixed with an equal volume of Freund's complete adjuvant was injected subcutaneously at multiple sites on the back for immunostimulation. The first immunization was performed by subcutaneous injection of 1 mg of KLH-polypeptide thoroughly mixed with an equal volume of Freund's complete adjuvant at multiple sites on the back and inner thigh. Booster immunization was performed for 4 times at an interval of 2 weeks, and the antigen of the booster immunization was 0.5 mg of KLH-polypeptide thoroughly mixed with an equal volume of Freund's incomplete adjuvant. Blood was collected 2 weeks after the last immunization and serum was isolated.

Seventeen HPV pseudoviruses were used to detect the titers of neutralizing antibodies in the immune serum, and the results were shown in Table 2. The 59RG-1 epitope peptide had the best immune activity, and its antiserum could neutralize all 17 types used for detection, of which the titers of neutralizing antibodies of HPV45, -59, -16 were all above 103, and the titers of neutralizing antibodies of HPV5, -31, -18, -39, -68, -57 were between 500 and 1000. It was worth noticing that the 59RG-1 epitope peptide antiserum had high levels of neutralizing antibodies against the five α7-HPV used for detection.

Methods of polypeptide synthesis, pseudovirus preparation and pseudoviral neutralization experiments were all publicly available, for example, the patents CN 104418942A and 108676057A.

**Table 1: Sequences of different types of RG-1 epitope peptides synthesized**

| Type | Sequence of synthetic peptide | SEQ ID No. |
|---|---|---|
| HPV35 | TQLYRTCKAAGTCPPDVIPKVEG | 44 |
| HPV39 | STLYRTCKQSGTCPPDVVDKVEG | 45 |
| HPV51 | TQLYSTCKAAGTCPPDVVNKVEG | 46 |
| HPV53 | TQLYQTCKQSGTCPEDVINKIEH | 47 |
| HPV56 | TQLYKTCKLSGTCPEDVVNKIEQ | 48 |
| HPV59 | LYKTCKQAGTCP SDVIN KVEGTT | 49 |
| HPV68 | STLYKTCKQSGTCPPDVINKVEG | 50 |
| HPV82 | TQLYSTCKAAGTCPPDVIPKVKG | 51 |

**Table 2: Titers of serum neutralizing antibodies induced by different RG1-KLH conjugated peptides in rabbits**

| | | | 35R G-1 | 39R G-1 | 51R G-1 | 53R G-1 | 56R G-1 | 59R G-1 | 68R G-1 | 82R G-1 |
|---|---|---|---|---|---|---|---|---|---|---|
| Average titer of neutralizing antibodies | α7 subgenus | HPV 18 | ND* | ND | ND | ND | 50 | 603 | 25 | 100 |
| | | HPV 39 | ND | 25 | ND | ND | 100 | 575 | 100 | 400 |
| | | HPV 45 | 25 | 25 | 25 | ND | 1200 | 1850 | 1600 | 400 |
| | | HPV 59 | ND | ND | ND | 100 | 25 | 1800 | ND | ND |
| | | HPV 68 | ND | ND | ND | ND | 75 | 525 | 425 | 100 |
| | α9 subgenus | HPV 16 | ND | ND | ND | ND | ND | 1063 | ND | 50 |
| | | HPV 31 | ND | ND | ND | ND | ND | 615 | ND | 25 |
| | | HPV 33 | ND | ND | ND | ND | ND | 118 | ND | 25 |
| | | HPV 35 | 25 | ND | ND | ND | ND | 240 | ND | 100 |
| | | HPV 52 | ND | ND | ND | ND | ND | 128 | ND | 50 |
| | | HPV 58 | ND | ND | ND | ND | 25 | 255 | ND | 100 |
| | α10 subgenus | HPV 6 | ND | ND | ND | ND | 25 | 68 | ND | 50 |
| | | HPV 11 | ND | 25 | ND | 25 | ND | 118 | 25 | 50 |
| | α4 subgenus | HPV 2 | 25 | 125 | ND | 50 | ND | 118 | ND | 50 |
| | | HPV 27 | 50 | 50 | 25 | 50 | 50 | 140 | 50 | 25 |
| | | HPV 57 | 75 | 50 | 50 | 50 | 75 | 515 | 50 | 125 |
| | β1 subgenus | HPV 5 | 50 | 50 | 25 | ND | 50 | 950 | 200 | 225 |

### Example 2: Synthesis of chimeric L1 protein genes and construction of expression vectors

There were 26 chimeric L1 proteins, namely:
(1) Chimeric L1 protein 18L1DE137-138/59dES: the backbone was full-length HPV type 18 L1 protein (the sequence was as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES chimeric protein was as shown in SEQ ID No. 7. The polynucleotide sequence encoding 18L1DE137-138/59dES was optimized with *E*. *coli* codons and constructed by whole-gene synthesis;
(2) Chimeric L1 protein 18L1DE137-138/59dE: the backbone was full-length HPV type 18 L1 protein (the sequence was as shown in SEQ ID No. 1), where the polypeptide of aa. 16-35 of HPV type 59 L2 protein (as shown in SEQ ID No. 5) was directly inserted at the aa. 137/138 site of its DE loop. The amino acid sequence of 18L1DE137-138/59dE chimeric protein was as shown in SEQ ID No. 9. The polynucleotide sequence encoding 18L1DE137-138/59dE was optimized with *E. coli* codons and constructed by whole-gene synthesis;
(3) Chimeric L1 protein 18L1h4432-433/59dE: the backbone was full-length HPV type 18 L1 protein (the sequence was as shown in SEQ ID No. 1), where the polypeptide of aa. 17-37 of HPV type 59 L2 protein (as shown in SEQ ID No. 3) was directly inserted at the aa. 432/433 site of its h4 region. The amino acid sequence of 18L1h4432-433/59dE chimeric protein was as shown in SEQ ID No. 11. The polynucleotide sequence encoding 18L1h4432-433/59dE was optimized with *E*. *coli* codons and constructed by whole-gene synthesis;
(4) Chimeric L1 protein 18L1h4434-435/59dE: the backbone was full-length HPV type 18 L1 protein (the sequence was as shown in SEQ ID No. 1), where the polypeptide of aa. 17-37 of HPV type 59 L2 protein (as shown in SEQ ID No. 3) was directly inserted at the aa. 434/435 site of its h4 region. The amino acid sequence of 18L1h4434-435/59dE chimeric protein was as shown in SEQ ID No. 13. The polynucleotide sequence encoding 18L1h4434-435/59dE was optimized with *E*. *coli* codons and constructed by whole-gene synthesis;
(5) Chimeric L1 protein 18L1DE134-135/59dES: the backbone was full-length HPV type 18 L1 protein (the sequence was as shown in SEQ ID No. 1), where the polypeptide of aa. 17-32 of HPV type 59 L2 protein containing a GP linker at N-terminus and a P linker at C-terminus (i.e., adding glycine-proline at N-terminus and adding proline at C-terminus of the sequence as shown in SEQ ID No. 4) was directly inserted at the aa. 134/135 site of its DE loop. The amino acid sequence of 18L1DE134-135/59dES chimeric protein was as shown in SEQ ID No. 15. The polynucleotide sequence encoding 18L1DE134-135/59dES was optimized with *E. coli* codons and constructed by whole-gene synthesis;
(6) Chimeric L1 protein 18L1DE134-135/59dE: the backbone was full-length HPV type 18 L1 protein (the sequence was as shown in SEQ ID No. 1), where the polypeptide of aa. 16-37 of HPV type 59 L2 protein containing a GP linker at N-terminus and a P linker at C-terminus (i.e., adding glycine-proline at N-terminus and adding proline at C-terminus of the sequence as shown in SEQ ID No. 6) was directly inserted at the aa. 134/135 site of its DE loop. The amino acid sequence of 18L1DE134-135/59dE chimeric protein was as shown in SEQ ID No. 17. The polynucleotide sequence encoding 18L1DE134-135/59dES was optimized with *E. coli* codons and constructed by whole-gene synthesis;
(7) Chimeric L1 protein 18L1DE131-138/59dE: the backbone was full-length HPV type 18 L1 protein (the sequence was as shown in SEQ ID No. 1), where the region of aa. 132-137 was deleted, and the polypeptide of aa. 17-37 of HPV type 59 L2 protein containing a GP linker at N-terminus was fused between aa. 131/138 (insertion at the region of aa. 132-137 of the HPV type 18 L1 protein by non-isometric substitution). The amino acid sequence of the inserted fragment was the sequence as shown in SEQ ID No. 3 with glycine-proline added at N-terminus, and the amino acid sequence of 18L1DE131-138/59dE chimeric protein was as shown in SEQ ID No. 19. The poly- nucleotide sequence encoding 18L1DE131-138/59dE was optimized with *E*. *coli* codons and constructed by whole-gene synthesis;
(8) Chimeric L1 protein 18L1DE121-124/59dE: the backbone was full-length HPV type 18 L1 protein (the sequence was as shown in SEQ ID No. 1), where the region of aa. 122-123 was deleted, and the polypeptide of aa. 17-37 of HPV type 59 L2 protein was fused between aa. 121/124 (insertion at the region of aa. 122-133 of the HPV type 18 L1 protein by non-isometric substitution). The amino acid sequence of the inserted fragment was as shown in SEQ ID No. 3, and the amino acid sequence of 18L1DE121-124/59dE chimeric protein was as shown in SEQ ID No. 21. The polynucleotide sequence encoding 18L1DE121-124/59dE was optimized with *E. coli* codons and constructed by whole-gene synthesis;
(9) Chimeric L1 protein 18L1h4431-433/59dE: the backbone was full-length HPV type 18 L1 protein (the sequence was as shown in SEQ ID No. 1), where aa. 432 was deleted, and the polypeptide of aa. 17-37 of HPV type 59 L2 protein was fused between aa. 431/433 (insertion at the region of aa. 431-433 of the HPV type 18 L1 protein by non-isometric substitution). The amino acid sequence of the inserted fragment was as shown in SEQ ID No. 3, and the amino acid sequence of 18L1h4431-433/59dE chimeric protein was as shown in SEQ ID No. 23. The polynucleotide sequence encoding 18L1h4431-433/59dE was optimized with *E*. *coli* codons and constructed by whole-gene synthesis;
(10) Chimeric L1 protein 18L1h4432-435/59dE: the backbone was full-length HPV type 18 L1 protein (the sequence was as shown in SEQ ID No. 1), where the region of aa. 433-434 was deleted, and the polypeptide of aa. 17-37 of HPV type 59 L2 protein was fused between aa. 432/435 (insertion at the region of aa. 432-435 of the HPV type 18 L1 protein by non-isometric substitution). The amino acid sequence of the inserted fragment was as shown in SEQ ID No. 3, and the amino acid sequence of 18L1h4432-435/59dE chimeric protein was as shown in SEQ ID No. 25. The polynucleotide sequence encoding 18L1h4433-434/59dE was optimized with *E*. *coli* codons and constructed by whole-gene synthesis;
(11) Chimeric L1 protein 18L1ΔCDE137-138/59dES: the backbone was the HPV type 18 L1 protein with 32-amino acid truncation at C-terminus (the sequence of SEQ ID No. 1 with 32-amino acid truncation at C-terminus), where the polypeptide of aa. 17-32 of HPV type 59 L2 protein (as shown in SEQ ID No. 4) was directly inserted at the aa. 137/138 site of its DE loop. The amino acid sequence of 18L1ΔCDE137-138/59dES chimeric protein was as shown in SEQ ID No. 8. The polynucleotide sequence encoding 18L1ΔCDE137-138/59dES was optimized with insect cell codons and constructed by whole-gene synthesis, and its nucleotide sequence was as shown in SEQ ID No. 33;
(12) Chimeric L1 protein 18L1ΔCDE137-138/59dE: the backbone was the HPV type 18 L1 protein with 32-amino acid truncation at C-terminus (the sequence of SEQ ID No. 1 with 32-amino acid truncation at C-terminus), where the polypeptide of aa. 16-35 of HPV type 59 L2 protein (as shown in SEQ ID No. 5) was directly inserted at the aa. 137/138 site of its DE loop. The amino acid sequence of 18L1ΔCDE137-138/59dE chimeric protein was as shown in SEQ ID No. 10. The polynucleotide sequence encoding 18L1ΔCDE137-138/59dE was optimized with insect cell codons and constructed by whole-gene synthesis, and its nucleotide sequence was as shown in SEQ ID No. 34;
(13) Chimeric L1 protein 18L1ΔCh4432-433/59dE: the backbone was the HPV type 18 L1 protein with 32-amino acid truncation at C-terminus (the sequence of SEQ ID No. 1 with 32-amino acid truncation at C-terminus), where the polypeptide of aa. 17-37 of HPV type 59 L2 protein (as shown in SEQ ID No. 3) was directly inserted at the aa. 432/433 site of its h4 region. The amino acid sequence of 18L1ΔCh4432-433/59dE chimeric protein was as shown in SEQ ID No. 12. The polynucleotide sequence encoding 18L1ΔCh4432-433/59dE was optimized with insect cell codons and constructed by whole-gene synthesis;
(14) Chimeric L1 protein 18L1ΔCh4434-435/59dE: the backbone was the HPV type 18 L1 protein with 32-amino acid truncation at C-terminus (the sequence of SEQ ID No. 1 with 32-amino acid truncation at C-terminus), where the polypeptide of aa. 17-37 of HPV type 59 L2 protein (as shown in SEQ ID No. 3) was directly inserted at the aa. 434/435 site of its h4 region. The amino acid sequence of 18L1ΔCh4434-435/59dE chimeric protein was as shown in SEQ ID No. 14. The polynucleotide sequence encoding 18L1ΔCh4434-435/59dE was optimized with insect cell codons and constructed by whole-gene synthesis;
(15) Chimeric L1 protein 18L1ΔCDE134-135/59dES: the backbone was the HPV type 18 L1 protein with 32-amino acid truncation at C-terminus (the sequence of SEQ ID No. 1 with 32-amino acid truncation at C-terminus), where the polypeptide of aa. 17-32 of HPV type 59 L2 protein containing a GP linker at N-terminus and a P linker at C-terminus (i.e., adding glycine-proline at N-terminus and adding proline at C-terminus of the sequence as shown in SEQ ID No. 4) was directly inserted at the aa. 134/135 site of its DE loop. The amino acid sequence of 18L1ΔCDE134-135/59dES chimeric protein was as shown in SEQ ID No. 16. The polynucleotide sequence encoding 18L1ΔCDE134-135/59dES was optimized with insect cell codons and constructed by whole-gene synthesis, and its nucleotide sequence was as shown in SEQ ID No. 35;
(16) Chimeric L1 protein 18L1ΔCDE134-135/59dE: the backbone was the HPV type 18 L1 protein with 32-amino acid truncation at C-terminus (the sequence of SEQ ID No. 1 with 32-amino acid truncation at C-terminus), where the polypeptide of aa. 16-37 of HPV type 59 L2 protein containing a GP linker at N-terminus and a P linker at C-terminus (i.e., adding glycine-proline at N-terminus and adding proline at C-terminus of the sequence as shown in SEQ ID No. 6) was directly inserted at the aa. 134/135 site of its DE loop. The amino acid sequence of 18L1ΔCDE134-135/59dE chimeric protein was as shown in SEQ ID No. 18. The polynucleotide sequence encoding 18L1ΔCDE134-135/59dE was optimized with insect cell codons and constructed by whole-gene synthesis, and its nucleotide sequence was as shown in SEQ ID No. 36;
(17) Chimeric L1 protein 18L1ΔCDE131-138/59dE: the backbone was the HPV type 18 L1 protein with 32-amino acid truncation at C-terminus (the sequence of SEQ ID No. 1 with 32-amino acid truncation at C-terminus), where the region of aa. 132-137 was deleted, and the polypeptide of aa. 17-37 of HPV type 59 L2 protein containing a GP linker at N-terminus was fused between aa. 131/138 (insertion at the region of aa. 132-137 of the HPV type 18 L1 protein by non-isometric substitution). The amino acid sequence of the inserted fragment was the sequence as shown in SEQ ID No. 3 with glycine-proline added at N-terminus, and the amino acid sequence of 18L1 DE131-138/59dE chimeric protein was as shown in SEQ ID No. 20. The polynucleotide sequence encoding 18L1DE131-138/59dE was optimized with insect cell codons and constructed by whole-gene synthesis, and its nucleotide sequence was as shown in SEQ ID No. 37;
(18) Chimeric L1 protein 18L1ΔCDE121-124/59dE: the backbone was the HPV type 18 L1 protein with 32-amino acid truncation at C-terminus (the sequence of SEQ ID No. 1 with 32-amino acid truncation at C-terminus), where the region of aa. 122-123 was deleted, and the polypeptide of aa. 17-37 of HPV type 59 L2 protein was fused between aa. 121/124 (insertion at the region of aa. 122-133 of the HPV type 18 L1 protein by non-isometric substitution). The amino acid sequence of the inserted fragment was as shown in SEQ ID No. 3, and the amino acid sequence of 18L1ΔCDE121-124/59dE chimeric protein was as shown in SEQ ID No. 22. The polynucleotide sequence encoding 18L1ΔCDE121-124/59dE was optimized with insect cell codons and constructed by whole-gene synthesis;
(19) Chimeric L1 protein 18L1ΔCh4431-433/59dE: the backbone was the HPV type 18 L1 protein with 32-amino acid truncation at C-terminus (the sequence of SEQ ID No. 1 with 32-amino acid truncation at C-terminus), where aa. 432 was deleted, and the polypeptide of aa. 17-37 of HPV type 59 L2 protein was fused between aa. 431/433 (insertion at the region of aa. 431-433 of the HPV type 18 L1 protein by non-isometric substitution). The amino acid sequence of the inserted fragment was as shown in SEQ ID No. 3, and the amino acid sequence of 18L1ΔCh4431-433/59dE chimeric protein was as shown in SEQ ID No. 24. The polynucleotide sequence encoding 18L1ΔCh4431-433/59dE was optimized with insect cell codons and constructed by whole-gene synthesis;
(20) Chimeric L1 protein 18L1ΔCh4432-435/59dE: the backbone was the HPV type 18 L1 protein with 32-amino acid truncation at C-terminus (the sequence of SEQ ID No. 1 with 32-amino acid truncation at C-terminus), where the region of aa. 433-434 was deleted, and the polypeptide of aa. 17-37 of HPV type 59 L2 protein was fused between aa. 432/435 (insertion at the region of aa. 432-435 of the HPV type 18 L1 protein by non-isometric substitution). The amino acid sequence of the inserted fragment was as shown in SEQ ID No. 3, and the amino acid sequence of 18L1ΔCh4432-435/59dE chimeric protein was as shown in SEQ ID No. 26. The polynucleotide sequence encoding 18L1ΔCh4432-435/59dE was optimized with insect cell codons and constructed by whole-gene synthesis;
(21) Chimeric L1 protein 18L1DE137-138/59dES-mut1: the backbone was the mutant mut1 of full-length HPV type 18 L1 protein (i.e., the mutant with amino acid substitutions of amino acids 477, 478, 484, 496, 499, 504, 506 to glycine (G) and amino acids 485, 500, 502 to serine (S) in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut1 chimeric protein was as shown in SEQ ID No. 27. The polynucleotide sequence encoding 18L1DE137-138/59dES-mut1 was optimized with E. *coli* codons or insect cell codons and constructed by whole-gene synthesis, wherein the polynucleotide sequence encoding 18L1DE137-138/59dES-mut1 optimized with insect cell codons was as shown in SEQ ID No. 38;
(22) Chimeric L1 protein 18L1DE137-138/59dES-mut2: the backbone was the mutant mut2 of full-length HPV type 18 L1 protein (i.e., the mutant with amino acid substitutions of amino acids 477, 478, 485, 496, 499, 504, 506 to glycine and amino acids 486, 500, 502 to serine in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut2 chimeric protein was as shown in SEQ ID No. 28. The polynucleotide sequence encoding 18L1DE137-138/59dES-mut2 was optimized with E. *coli* codons or insect cell codons and constructed by whole-gene synthesis, wherein the polynucleotide sequence encoding 18L1DE137-138/59dES-mut2 optimized with insect cell codons was as shown in SEQ ID No. 39;
(23) Chimeric L1 protein 18L1DE137-138/59dES-mut3: the backbone was the mutant mut3 of full-length HPV type 18 L1 protein (i.e., the mutant with amino acid substitutions of amino acids 477, 478, 484, 496, 499, 502, 506 to glycine, amino acids 485, 500 to serine and amino acid 504 to aspartate in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut3 chimeric protein was as shown in SEQ ID No. 29. The polynucleotide sequence encoding 18L1DE137-138/59dES-mut3 was optimized with *E. coli* codons or insect cell codons and constructed by whole-gene synthesis, wherein the polynucleotide sequence encoding 18L1DE137-138/59dES-mut3 optimized with insect cell codons was as shown in SEQ ID No. 40;
(24) Chimeric L1 protein 18L1DE137-138/59dES-mut4: the backbone was the mutant mut4 of full-length HPV type 18 L1 protein (i.e., the mutant with amino acid substitutions of amino acids 477, 478, 485, 496, 502, 506 to glycine, amino acids 486, 500 to serine and amino acids 499, 504 to aspartate in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut4 chimeric protein was as shown in SEQ ID No. 30. The polynucleotide sequence encoding 18L1DE137-138/59dES-mut4 was optimized with *E. coli* codons or insect cell codons and constructed by whole-gene synthesis, wherein the polynucleotide sequence encoding 18L1DE137-138/59dES-mut4 optimized with insect cell codons was as shown in SEQ ID No. 41;
(25) Chimeric L1 protein 18L1DE137-138/59dES-mut5: the backbone was the mutant mut5 of full-length HPV type 18 L1 protein (i.e., the mutant with amino acid substitutions of amino acids 477, 484, 496, 499, 504, 506 to glycine and amino acids 485, 500, 502 to serine in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut5 chimeric protein was as shown in SEQ ID No. 31. The polynucleotide sequence encoding 18L1DE137-138/59dES-mut5 was optimized with *E*. *coli* codons or insect cell codons and constructed by whole-gene synthesis, wherein the polynucleotide sequence encoding 18L1DE137-138/59dES-mut5 optimized with insect cell codons was as shown in SEQ ID No. 42;
(26) Chimeric L1 protein 18L1DE137-138/59dES-mut6: the backbone was the mutant mut6 of full-length HPV type 18 L1 protein (i.e., the mutant with amino acid substitutions of amino acids 477, 485, 496, 499, 504, 506 to glycine and amino acids 486, 500, 502 to serine in the amino acid sequence as shown in SEQ ID No. 1), where the polypeptide of aa. 17-32 of HPV type 59 L2 protein (as shown in SEQ ID No. 4) was directly inserted at the aa. 137/138 site of its DE loop. The amino acid sequence of 18L1DE137-138/59dES-mut6 chimeric protein was as shown in SEQ ID No. 32. The polynucleotide sequence encoding 18L1DE137-138/59dES-mut6 was optimized with *E*. *coli* codons or insect cell codons and constructed by whole-gene synthesis, wherein the polynucleotide sequence encoding 18L1DE137-138/59dES-mut5 optimized with insect cell codons was as shown in SEQ ID No. 43.

The chimeric protein genes optimized with *E*. *coli* codons were digested by Ndel/Xhol and inserted into the commercial expression vector pET22b (produced by Novagen), respectively. The chimeric protein genes optimized with insect cell codons were digested by BamHI/EcoRI and inserted into the commercial expression vector pFastBac1 (produced by Invitrogen), respectively. Expression vectors comprising the chimeric protein genes were obtained. There were a total of 16 expression vectors for *E. coli,* namely: pET22b-18L1DE137-138/59dES, pET22b-18L1DE137-138/59dE, pET22b-18L1h4432-433/59dE, pET22b-18L1h4434-435/59dE, pET22b-18L1DE134-135/59dES, pET22b-18L1DE134-135/59dE, pET22b-18L1DE131-138/59dE, pET22b-18L1DE121-124/59dE, pET22b-18L1h4431-433/59dE, pET22b-18L1h4432-435/59dE, pET22b-18L1DE137-138/59dES-mut1, pET22b-18L1DE137-138/59dES-mut2, pET22b-18L1DE137-138/59dES-mut3, pET22b-18L1DE137-138/59dES-mut4, pET22b-18L1DE137-138/59dES-mut5, pET22b-18L1DE137-138/59dES-mut6. There were a total of 16 expression vectors for insect cells, namely: pFastBac1-18L1ΔCDE137-138/59dES, pFastBac1-18L1ΔCDE137-138/59dE, pFastBac1-18L1ΔCh4432-433/59dE, pFastBac1-18L1ΔCh4434-435/59dE, pFastBac1-18L1ΔCDE134-135/59dES, pFastBac1-18L1ΔCDE134-135/59dE, pFastBac1-18L1ΔCDE131-138/59dE, pFastBac1-18L1ΔCDE121-124/59dE, pFastBac1-18L1ΔCh4431-433/59dE, pFastBac1-18L1ΔCh4432-435/59dE, pFastBac1-18L1DE137-138/59dES-mut1, pFastBac1-18L1DE137-138/59dES-mut2, pFastBac1-18L1DE137-138/59dES-mut3, pFastBac1-18L1DE137-138/59dES-mut4, pFastBac1-18L1DE137-138/59dES-mut5, pFastBac1-18L1DE137- 138/59dES-mut6. The above methods of enzyme digestion, ligation and construction of clones were all well known, for example, the patent CN 101293918 B.

The amino acid sequences of the polypeptides used in the present invention were shown as follows:
Full-length amino acids of HPV type 18 L1 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDAAPAENKD PYDKLKFWNV DLKEKFSLDL DQYPLGRKFL VQAGLRRKPT
IGPRKRSAPS ATTSSKPAKR VRVRARK SEQ ID NO. 1
Full-length amino acid sequence of HPV type 59 L2 
MVSHRAARRK RASATDLYKT CKQAGTCPSD VINKVEGTTL ADKILQWTSL GIFLGGLGIG
TGSGTGGRTG YIPLGGRTNT IVDVSPAKPP VVIEPVGPTD PSIVTLVEDS SVITSGAPAP
TFTGTSGFEI STSSTTTPAV LDITPTSSVQ ISSSSFINPA FTDPSVIEVP QTGEISGNIL
ISTPTSGAHG YEEIPMQTFA TEGTGLEPIS STPNPTVRRV AGPRLYSRAN QQVRVSDANF
LTRPSTFVTY DNPAYDPIDT TLTFDPSSEV PDPDFMDIVR LHRPALTSRR STVRFSRLGQ
RATMFTRSGK QIGARVHFYH DISPIPHAEN IELQPLVSSQ AATDDIYDIY ADITDEAPTS
TANTAFTIPK SSFQSLSLTR SASSTFSNVT VPLATAWDVP VNTGPDIVLP NTNIVGPTYS
TTPFTTIQSI NIEGTNYFLW PIYYFLPRKR KRVPYFFTDG SMAF SEQ ID NO. 2
aa. 17-37 of HPV type 59 L2
   LYKTCKQAGT CPSDVINKVE G SEQ ID NO. 3
aa. 17-32 of HPV type 59 L2
   LYKTCKQAGT CPSDVI SEQ ID NO. 4
aa. 16-35 of HPV type 59 L2
   DLYKTCKQAG TCPSDVINKV SEQ ID NO. 5
aa. 16-37 of HPV type 59 L2
   DLYKTCKQAG TCPSDVINKV EG SEQ ID NO. 6
18L1DE137-138/59dES 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRRKPTIGPR KRSAPSATTS SKPAKRVRVR ARK SEQ ID NO. 7
18L1ΔCDE137-138/59dES 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
 
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG L SEQ ID No.8
18L1DE137-138/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSDLY KTCKQAGTCP SDVINKVNVS EDVRDNVSVD YKQTQLCILG
CAPAIGEHWA KGTACKSRPL SQGDCPPLEL KNTVLEDGDM VDTGYGAMDF STLQDTKCEV
PLDICQSICK YPDYLQMSAD PYGDSMFFCL RREQLFARHF WNRAGTMGDT VPQSLYIKGT
GMRASPGSCV YSPSPSGSIV TSDSQLFNKP YWLHKAQGHN NGVCWHNQLF VTVVDTTRST
NLTICASTQS PVPGQYDATK FKQYSRHVEE YDLQFIFQLC TITLTADVMS YIHSMNSSIL
EDWNFGVPPP PTTSLVDTYR FVQSVAITCQ KDAAPAENKD PYDKLKFWNV DLKEKFSLDL
DQYPLGRKFL VQAGLRRKPT IGPRKRSAPS ATTSSKPAKR VRVRARK SEQ ID No.9
18L1ΔCDE137-138/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSDLY KTCKQAGTCP SDVINKVNVS EDVRDNVSVD YKQTQLCILG
CAPAIGEHWA KGTACKSRPL SQGDCPPLEL KNTVLEDGDM VDTGYGAMDF STLQDTKCEV
PLDICQSICK YPDYLQMSAD PYGDSMFFCL RREQLFARHF WNRAGTMGDT VPQSLYIKGT
GMRASPGSCV YSPSPSGSIV TSDSQLFNKP YWLHKAQGHN NGVCWHNQLF VTVVDTTRST
NLTICASTQS PVPGQYDATK FKQYSRHVEE YDLQFIFQLC TITLTADVMS YIHSMNSSIL
EDWNFGVPPP PTTSLVDTYR FVQSVAITCQ KDAAPAENKD PYDKLKFWNV DLKEKFSLDL
DQYPLGRKFL VQAGL SEQ ID No.10
18L1h4432-433/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDLYKTCKQA GTCPSDVINK VEGAAPAENK DPYDKLKFWN VDLKEKFSLD
LDQYPLGRKF LVQAGLRRKP TIGPRKRSAP SATTSSKPAK RVRVRARK SEQ ID No.11
18L1ΔCh4432-433/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDLYKTCKQA GTCPSDVINK VEGAAPAENK DPYDKLKFWN VDLKEKFSLD
LDQYPLGRKF LVQAGL SEQ ID No.12
18L1h4434-435/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDAALYKTCK QAGTCPSDVI NKVEGPAENK DPYDKLKFWN VDLKEKFSLD
LDQYPLGRKF LVQAGLRRKP TIGPRKRSAP SATTSSKPAK RVRVRARK SEQ ID No.13
18L1ΔCh4434-435/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDAALYKTCK QAGTCPSDVI NKVEGPAENK DPYDKLKFWN VDLKEKFSLD
LDQYPLGRKF LVQAGL SEQ ID No.14
18L1DE134-135/59dES 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAGPLYKT CKQAGTCPSD VIPATSNVSE DVRDNVSVDY KQTQLCILGC
APAIGEHWAK GTACKSRPLS QGDCPPLELK NTVLEDGDMV DTGYGAMDFS TLQDTKCEVP
LDICQSICKY PDYLQMSADP YGDSMFFCLR REQLFARHFW NRAGTMGDTV PQSLYIKGTG
MRASPGSCVY SPSPSGSIVT SDSQLFNKPY WLHKAQGHNN GVCWHNQLFV TVVDTTRSTN
LTICASTQSP VPGQYDATKF KQYSRHVEEY DLQFIFQLCT ITLTADVMSY IHSMNSSILE
DWNFGVPPPP TTSLVDTYRF VQSVAITCQK DAAPAENKDP YDKLKFWNVD LKEKFSLDLD
QYPLGRKFLV QAGLRRKPT IGPRKRSAPS ATTSSKPAKR VRVRARK SEQ ID No.15
18L1ΔCDE134-135/59dES 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAGPLYKT CKQAGTCPSD VIPATSNVSE DVRDNVSVDY KQTQLCILGC
APAIGEHWAK GTACKSRPLS QGDCPPLELK NTVLEDGDMV DTGYGAMDFS TLQDTKCEVP
LDICQSICKY PDYLQMSADP YGDSMFFCLR REQLFARHFW NRAGTMGDTV PQSLYIKGTG
MRASPGSCVY SPSPSGSIVT SDSQLFNKPY WLHKAQGHNN GVCWHNQLFV TVVDTTRSTN
LTICASTQSP VPGQYDATKF KQYSRHVEEY DLQFIFQLCT ITLTADVMSY IHSMNSSILE
DWNFGVPPPP TTSLVDTYRF VQSVAITCQK DAAPAENKDP YDKLKFWNVD LKEKFSLDLD
QYPLGRKFLV QAGL SEQ ID No.16
18L1DE134-135/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAGPDLYK TCKQAGTCPS DVINKVEGPA TSNVSEDVRD NVSVDYKQTQ
LCILGCAPAI GEHWAKGTAC KSRPLSQGDC PPLELKNTVL EDGDMVDTGY GAMDFSTLQD
TKCEVPLDIC QSICKYPDYL QMSADPYGDS MFFCLRREQL FARHFWNRAG TMGDTVPQSL
 
YIKGTGMRAS PGSCVYSPSP SGSIVTSDSQ LFNKPYWLHK AQGHNNGVCW HNQLFVTVVD
TTRSTNLTIC ASTQSPVPGQ YDATKFKQYS RHVEEYDLQF IFQLCTITLT ADVMSYIHSM
NSSILEDWNF GVPPPPTTSL VDTYRFVQSV AITCQKDAAP AENKDPYDKL KFWNVDLKEK
FSLDLDQYPL GRKFLVQAGL RRKPT IGPRKRSAPS ATTSSKPAKR VRVRARK
SEQ ID No.17
18L1ΔCDE134-135/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAGPDLYK TCKQAGTCPS DVINKVEGPA TSNVSEDVRD NVSVDYKQTQ
LCILGCAPAI GEHWAKGTAC KSRPLSQGDC PPLELKNTVL EDGDMVDTGY GAMDFSTLQD
TKCEVPLDIC QSICKYPDYL QMSADPYGDS MFFCLRREQL FARHFWNRAG TMGDTVPQSL
YIKGTGMRAS PGSCVYSPSP SGSIVTSDSQ LFNKPYWLHK AQGHNNGVCW HNQLFVTVVD
TTRSTNLTIC ASTQSPVPGQ YDATKFKQYS RHVEEYDLQF IFQLCTITLT ADVMSYIHSM
NSSILEDWNF GVPPPPTTSL VDTYRFVQSV AITCQKDAAP AENKDPYDKL KFWNVDLKEK
FSLDLDQYPL GRKFLVQAGL SEQ ID No.18
18L1DE131-138/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SGPLYKTCKQ AGTCPSDVIN KVEGNVSEDV RDNVSVDYKQ TQLCILGCAP
AIGEHWAKGT ACKSRPLSQG DCPPLELKNT VLEDGDMVDT GYGAMDFSTL QDTKCEVPLD
ICQSICKYPD YLQMSADPYG DSMFFCLRRE QLFARHFWNR AGTMGDTVPQ SLYIKGTGMR
ASPGSCVYSP SPSGSIVTSD SQLFNKPYWL HKAQGHNNGV CWHNQLFVTV VDTTRSTNLT
ICASTQSPVP GQYDATKFKQ YSRHVEEYDL QFIFQLCTIT LTADVMSYIH SMNSSILEDW
NFGVPPPPTT SLVDTYRFVQ SVAITCQKDA APAENKDPYD KLKFWNVDLK EKFSLDLDQY
PLGRKFLVQA GL RRKPT IGPRKRSAPS ATTSSKPAKR VRVRARK SEQ ID No.19
18L1ΔCDE131-138/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SGPLYKTCKQ AGTCPSDVIN KVEGNVSEDV RDNVSVDYKQ TQLCILGCAP
AIGEHWAKGT ACKSRPLSQG DCPPLELKNT VLEDGDMVDT GYGAMDFSTL QDTKCEVPLD
ICQSICKYPD YLQMSADPYG DSMFFCLRRE QLFARHFWNR AGTMGDTVPQ SLYIKGTGMR
ASPGSCVYSP SPSGSIVTSD SQLFNKPYWL HKAQGHNNGV CWHNQLFVTV VDTTRSTNLT
ICASTQSPVP GQYDATKFKQ YSRHVEEYDL QFIFQLCTIT LTADVMSYIH SMNSSILEDW
NFGVPPPPTT SLVDTYRFVQ SVAITCQKDA APAENKDPYD KLKFWNVDLK EKFSLDLDQY
PLGRKFLVQA GL SEQ ID No.20
18L1DE121-124/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PLYKTCKQAG TCPSDVINKV EGNKLDDTES SHAATSNVSE DVRDNVSVDY KQTQLCILGC
APAIGEHWAK GTACKSRPLS QGDCPPLELK NTVLEDGDMV DTGYGAMDFS TLQDTKCEVP
LDICQSICKY PDYLQMSADP YGDSMFFCLR REQLFARHFW NRAGTMGDTV PQSLYIKGTG
MRASPGSCVY SPSPSGSIVT SDSQLFNKPY WLHKAQGHNN GVCWHNQLFV TVVDTTRSTN
LTICASTQSP VPGQYDATKF KQYSRHVEEY DLQFIFQLCT ITLTADVMSY IHSMNSSILE
DWNFGVPPPP TTSLVDTYRF VQSVAITCQK DAAPAENKDP YDKLKFWNVD LKEKFSLDLD
QYPLGRKFLV QAGLRRKPTI GPRKRSAPSA TTSSKPAKRV RVRARK SEQ ID No.21
18L1ΔCDE121-124/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PLYKTCKQAG TCPSDVINKV EGNKLDDTES SHAATSNVSE DVRDNVSVDY KQTQLCILGC
APAIGEHWAK GTACKSRPLS QGDCPPLELK NTVLEDGDMV DTGYGAMDFS TLQDTKCEVP
LDICQSICKY PDYLQMSADP YGDSMFFCLR REQLFARHFW NRAGTMGDTV PQSLYIKGTG
MRASPGSCVY SPSPSGSIVT SDSQLFNKPY WLHKAQGHNN GVCWHNQLFV TVVDTTRSTN
LTICASTQSP VPGQYDATKF KQYSRHVEEY DLQFIFQLCT ITLTADVMSY IHSMNSSILE
DWNFGVPPPP TTSLVDTYRF VQSVAITCQK DAAPAENKDP YDKLKFWNVD LKEKFSLDLD
QYPLGRKFLV QAGL SEQ ID No.22
18L1h4431-433/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KLYKTCKQAG TCPSDVINKV EGAAPAENKD PYDKLKFWNV DLKEKFSLDL
DQYPLGRKFL VQAGLRRKPT IGPRKRSAPS ATTSSKPAKR VRVRARK SEQ ID No.23
18L1ΔCh4431-433/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KLYKTCKQAG TCPSDVINKV EGAAPAENKD PYDKLKFWNV DLKEKFSLDL
DQYPLGRKFL VQAGL SEQ ID No.24
18L1h4432-435/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDLYKTCKQA GTCPSDVINK VEGPAENKDP YDKLKFWNVD LKEKFSLDLD
QYPLGRKFLV QAGLRRKPTI GPRKRSAPSA TTSSKPAKRV RVRARK    SEQ ID No.25
18L1ΔCh4432-435/59dE 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSNVS EDVRDNVSVD YKQTQLCILG CAPAIGEHWA KGTACKSRPL
SQGDCPPLEL KNTVLEDGDM VDTGYGAMDF STLQDTKCEV PLDICQSICK YPDYLQMSAD
 
PYGDSMFFCL RREQLFARHF WNRAGTMGDT VPQSLYIKGT GMRASPGSCV YSPSPSGSIV
TSDSQLFNKP YWLHKAQGHN NGVCWHNQLF VTVVDTTRST NLTICASTQS PVPGQYDATK
FKQYSRHVEE YDLQFIFQLC TITLTADVMS YIHSMNSSIL EDWNFGVPPP PTTSLVDTYR
FVQSVAITCQ KDLYKTCKQA GTCPSDVINK VEGPAENKDP YDKLKFWNVD LKEKFSLDLD
QYPLGRKFLV QAGL SEQ ID No.26
18L1DE137-138/59dES-mut1 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGGPTIGPG SRSAPSATTS SGPAGSVSVG AGK SEQ ID No.27
18L1DE137-138/59dES-mut2 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGGPTIGPR GSSAPSATTS SGPAGSVSVG AGK SEQ ID No.28
18L1DE137-138/59dES-mut3 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGGPTIGPG SRSAPSATTS SGPAGSVGVD AGK SEQ ID No.29
18L1DE137-138/59dES-mut4 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGGPTIGPR GSSAPSATTS SGPADSVGVD AGK SEQ ID No.30
18L1DE137-138/59dES-mut5 
MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGKPTIGPG SRSAPSATTS SGPAGSVSVG AGK SEQ ID No.31
18L1DE137-138/59dES-mut6

MALWRPSDNT VYLPPPSVAR VVNTDDYVTR TSIFYHAGSS RLLTVGNPYF RVPAGGGNKQ
DIPKVSAYQY RVFRVQLPDP NKFGLPDTSI YNPETQRLVW ACAGVEIGRG QPLGVGLSGH
PFYNKLDDTE SSHAATSLYK TCKQAGTCPS DVINVSEDVR DNVSVDYKQT QLCILGCAPA
IGEHWAKGTA CKSRPLSQGD CPPLELKNTV LEDGDMVDTG YGAMDFSTLQ DTKCEVPLDI
CQSICKYPDY LQMSADPYGD SMFFCLRREQ LFARHFWNRA GTMGDTVPQS LYIKGTGMRA
SPGSCVYSPS PSGSIVTSDS QLFNKPYWLH KAQGHNNGVC WHNQLFVTVV DTTRSTNLTI
CASTQSPVPG QYDATKFKQY SRHVEEYDLQ FIFQLCTITL TADVMSYIHS MNSSILEDWN
FGVPPPPTTS LVDTYRFVQS VAITCQKDAA PAENKDPYDK LKFWNVDLKE KFSLDLDQYP
LGRKFLVQAG LRGKPTIGPR GSSAPSATTS SGPAGSVSVG AGK SEQ ID No.32

The nucleotide sequences encoding the chimeric proteins of the present invention were shown as follows:
18L1ΔCDE137-138/59dES nt 
ATG GCT CTC TGG AGA CCC TCC GAT AAC ACA GTG TAC TTG CCC CCC CCC AGC
GTC GCC CGC GTC GTG AAC ACA GAC GAC TAC GTC ACC AGG ACC TCA ATC TTC
TAC CAC GCC GGT TCA AGC CGC CTG CTG ACC GTC GGC AAC CCC TAC TTC CGC
GTC CCC GCC GGT GGC GGT AAC AAA CAA GAC ATC CCC AAA GTC AGC GCC TAT
CAG TAC CGC GTG TTC CGC GTC CAA CTG CCC GAT CCC AAC AAG TTC GGC CTG
CCC GAC ACC TCC ATC TAC AAC CCC GAG ACC CAG AGG CTG GTC TGG GCA TGC
GCC GGC GTC GAG ATC GGT AGG GGC CAA CCC CTG GGC GTC GGT TTG TCC GGC
CAC CCC TTC TAC AAC AAG CTG GAC GAT ACC GAG TCC TCC CAC GCA GCA ACC
AGC CTG TAC AAG ACC TGC AAG CAG GCC GGT ACC TGC CCC TCC GAC GTC ATC
AAC GTC AGC GAA GAT GTC CGC GAT AAC GTC AGC GTG GAC TAC AAA CAA ACC
CAA CTG TGC ATC CTC GGT TGC GCA CCC GCC ATC GGC GAG CAT TGG GCC AAG
GGT ACC GCC TGC AAG AGC AGG CCC CTG AGC CAA GGT GAC TGT CCA CCC CTG
GAG TTG AAG AAT ACC GTC CTC GAG GAC GGC GAC ATG GTG GAC ACC GGC TAC
GGC GCA ATG GAT TTC TCC ACC CTG CAG GAC ACC AAG TGC GAA GTG CCC CTC
GAC ATC TGC CAA AGC ATC TGC AAG TAC CCC GAC TAC CTG CAG ATG AGC GCC
GAC CCC TAC GGC GAC TCC ATG TTC TTC TGT CTG AGA AGG GAA CAA TTG TTC
GCC CGC CAC TTC TGG AAC CGC GCC GGC ACC ATG GGC GAT ACC GTC CCC CAG
TCC CTG TAC ATC AAG GGT ACC GGC ATG AGG GCC AGC CCC GGT TCA TGC GTC
TAC AGC CCA AGC CCC TCC GGT AGC ATC GTC ACA AGC GAT TCC CAA CTC TTC
AAC AAG CCC TAC TGG CTG CAC AAA GCC CAA GGC CAC AAT AAC GGC GTC TGT
TGG CAC AAC CAG CTG TTC GTC ACC GTC GTG GAC ACA ACC AGG TCC ACA AAC
CTG ACC ATC TGC GCC AGC ACC CAA AGC CCC GTG CCC GGC CAG TAC GAC GCC
 
ACA AAG TTC AAA CAA TAC TCA CGC CAC GTC GAA GAG TAC GAC CTC CAA TTC
ATC TTC CAA CTC TGC ACC ATC ACC CTG ACC GCC GAC GTC ATG TCC TAC ATC
CAC TCC ATG AAC TCA TCC ATC CTG GAA GAC TGG AAT TTC GGC GTC CCA CCA
CCC CCC ACC ACC TCC CTC GTC GAC ACC TAC AGG TTC GTG CAG AGC GTC GCC
ATC ACA TGC CAG AAA GAC GCC GCC CCC GCC GAG AAC AAA GAC CCA TAC GAC
AAA CTG AAA TTC TGG AAC GTC GAC CTG AAA GAG AAA TTC AGC CTG GAT CTG
GAC CAG TAC CCA TTG GGC AGG AAG TTC CTC GTC CAG GCG GGT CTC TAA T
 SEQ ID No.33
18L1ΔCDE137-138/59dE nt 
ATG GCT CTC TGG AGA CCC TCC GAT AAC ACA GTG TAC TTG CCC CCC AGC
GTC GCC CGC GTC GTG AAC ACA GAC GAC TAC GTC ACC AGG ACC TCA ATC TTC
TAC CAC GCC GGT TCA AGC CGC CTG CTG ACC GTC GGC AAC CCC TAC TTC CGC
GTC CCC GCC GGT GGC GGT AAC AAA CAA GAC ATC CCC AAA GTC AGC GCC TAT
CAG TAC CGC GTG TTC CGC GTC CAA CTG CCC GAT CCC AAC AAG TTC GGC CTG
CCC GAC ACC TCC ATC TAC AAC CCC GAG ACC CAG AGG CTG GTC TGG GCA TGC
GCC GGC GTC GAG ATC GGT AGG GGC CAA CCC CTG GGC GTC GGT TTG TCC GGC
CAC CCC TTC TAC AAC AAG CTG GAC GAT ACC GAG TCC TCC CAC GCA GCA ACC
AGC GAC CTG TAC AAG ACC TGC AAG CAG GCC GGT ACC TGC CCC TCC GAC GTC
ATC AAC AAG GTC AAC GTC AGC GAA GAT GTC CGC GAT AAC GTC AGC GTG GAC
TAC AAA CAA ACC CAA CTG TGC ATC CTC GGT TGC GCA CCC GCC ATC GGC GAG
CAT TGG GCC AAG GGT ACC GCC TGC AAG AGC AGG CCC CTG AGC CAA GGT GAC
TGT CCA CCC CTG GAG TTG AAG AAT ACC GTC CTC GAG GAC GGC GAC ATG GTG
GAC ACC GGC TAC GGC GCA ATG GAT TTC TCC ACC CTG CAG GAC ACC AAG TGC
GAA GTG CCC CTC GAC ATC TGC CAA AGC ATC TGC AAG TAC CCC GAC TAC CTG
CAG ATG AGC GCC GAC CCC TAC GGC GAC TCC ATG TTC TTC TGT CTG AGA AGG
GAA CAA TTG TTC GCC CGC CAC TTC TGG AAC CGC GCC GGC ACC ATG GGC GAT
ACC GTC CCC CAG TCC CTG TAC ATC AAG GGT ACC GGC ATG AGG GCC AGC CCC
GGT TCA TGC GTC TAC AGC CCA AGC CCC TCC GGT AGC ATC GTC ACA AGC GAT
TCC CAA CTC TTC AAC AAG CCC TAC TGG CTG CAC AAA GCC CAA GGC CAC AAT
AAC GGC GTC TGT TGG CAC AAC CAG CTG TTC GTC ACC GTC GTG GAC ACA ACC
AGG TCC ACA AAC CTG ACC ATC TGC GCC AGC ACC CAA AGC CCC GTG CCC GGC
CAG TAC GAC GCC ACA AAG TTC AAA CAA TAC TCA CGC CAC GTC GAA GAG TAC
GAC CTC CAA TTC ATC TTC CAA CTC TGC ACC ATC ACC CTG ACC GCC GAC GTC
ATG TCC TAC ATC CAC TCC ATG AAC TCA TCC ATC CTG GAA GAC TGG AAT TTC
GGC GTC CCA CCA CCC CCC ACC ACC TCC CTC GTC GAC ACC TAC AGG TTC GTG
CAG AGC GTC GCC ATC ACA TGC CAG AAA GAC GCC GCC CCC GCC GAG AAC AAA
GAC CCA TAC GAC AAA CTG AAA TTC TGG AAC GTC GAC CTG AAA GAG AAA TTC
AGC CTG GAT CTG GAC CAG TAC CCA TTG GGC AGG AAG TTC CTC GTC CAG GCG
GGT CTC TAA T SEQ ID No.34
18L1ΔCDE134-135/59dES nt 
ATG GCT CTC TGG AGA CCC TCC GAT AAC ACA GTG TAC TTG CCC CCC CCC AGC
GTC GCC CGC GTC GTG AAC ACA GAC GAC TAC GTC ACC AGG ACC TCA ATC TTC
TAC CAC GCC GGT TCA AGC CGC CTG CTG ACC GTC GGC AAC CCC TAC TTC CGC
GTC CCC GCC GGT GGC GGT AAC AAA CAA GAC ATC CCC AAA GTC AGC GCC TAT
CAG TAC CGC GTG TTC CGC GTC CAA CTG CCC GAT CCC AAC AAG TTC GGC CTG
CCC GAC ACC TCC ATC TAC AAC CCC GAG ACC CAG AGG CTG GTC TGG GCA TGC
GCC GGC GTC GAG ATC GGT AGG GGC CAA CCC CTG GGC GTC GGT TTG TCC GGC
CAC CCC TTC TAC AAC AAG CTG GAC GAT ACC GAG TCC TCC CAC GCA GGA CCA
 
CTG TAC AAG ACC TGC AAG CAG GCC GGT ACC TGC CCC TCC GAC GTC ATC CCA
GCA ACC AGC AAC GTC AGC GAA GAT GTC CGC GAT AAC GTC AGC GTG GAC TAC
AAA CAA ACC CAA CTG TGC ATC CTC GGT TGC GCA CCC GCC ATC GGC GAG CAT
TGG GCC AAG GGT ACC GCC TGC AAG AGC AGG CCC CTG AGC CAA GGT GAC TGT
CCA CCC CTG GAG TTG AAG AAT ACC GTC CTC GAG GAC GGC GAC ATG GTG GAC
ACC GGC TAC GGC GCA ATG GAT TTC TCC ACC CTG CAG GAC ACC AAG TGC GAA
GTG CCC CTC GAC ATC TGC CAA AGC ATC TGC AAG TAC CCC GAC TAC CTG CAG
ATG AGC GCC GAC CCC TAC GGC GAC TCC ATG TTC TTC TGT CTG AGA AGG GAA
CAA TTG TTC GCC CGC CAC TTC TGG AAC CGC GCC GGC ACC ATG GGC GAT ACC
GTC CCC CAG TCC CTG TAC ATC AAG GGT ACC GGC ATG AGG GCC AGC CCC GGT
TCA TGC GTC TAC AGC CCA AGC CCC TCC GGT AGC ATC GTC ACA AGC GAT TCC
CAA CTC TTC AAC AAG CCC TAC TGG CTG CAC AAA GCC CAA GGC CAC AAT AAC
GGC GTC TGT TGG CAC AAC CAG CTG TTC GTC ACC GTC GTG GAC ACA ACC AGG
TCC ACA AAC CTG ACC ATC TGC GCC AGC ACC CAA AGC CCC GTG CCC GGC CAG
TAC GAC GCC ACA AAG TTC AAA CAA TAC TCA CGC CAC GTC GAA GAG TAC GAC
CTC CAA TTC ATC TTC CAA CTC TGC ACC ATC ACC CTG ACC GCC GAC GTC ATG
TCC TAC ATC CAC TCC ATG AAC TCA TCC ATC CTG GAA GAC TGG AAT TTC GGC
GTC CCA CCA CCC CCC ACC ACC TCC CTC GTC GAC ACC TAC AGG TTC GTG CAG
AGC GTC GCC ATC ACA TGC CAG AAA GAC GCC GCC CCC GCC GAG AAC AAA GAC
CCA TAC GAC AAA CTG AAA TTC TGG AAC GTC GAC CTG AAA GAG AAA TTC AGC
CTG GAT CTG GAC CAG TAC CCA TTG GGC AGG AAG TTC CTC GTC CAG GCG GGT
CTC TAA T                                                SEQ ID No.35
18L1ΔCDE134-135/59dE nt 
ATG GCT CTC TGG AGA CCC TCC GAT AAC ACA GTG TAC TTG CCC CCC CCC AGC
GTC GCC CGC GTC GTG AAC ACA GAC GAC TAC GTC ACC AGG ACC TCA ATC TTC
TAC CAC GCC GGT TCA AGC CGC CTG CTG ACC GTC GGC AAC CCC TAC TTC CGC
GTC CCC GCC GGT GGC GGT AAC AAA CAA GAC ATC CCC AAA GTC AGC GCC TAT
CAG TAC CGC GTG TTC CGC GTC CAA CTG CCC GAT CCC AAC AAG TTC GGC CTG
CCC GAC ACC TCC ATC TAC AAC CCC GAG ACC CAG AGG CTG GTC TGG GCA TGC
GCC GGC GTC GAG ATC GGT AGG GGC CAA CCC CTG GGC GTC GGT TTG TCC GGC
CAC CCC TTC TAC AAC AAG CTG GAC GAT ACC GAG TCC TCC CAC GCA GGA CCA
GAC CTG TAC AAG ACC TGC AAG CAG GCC GGT ACC TGC CCC TCC GAC GTC ATC
AAC AAG GTC GAA GGA CCA GCA ACC AGC AAC GTC AGC GAA GAT GTC CGC GAT
AAC GTC AGC GTG GAC TAC AAA CAA ACC CAA CTG TGC ATC CTC GGT TGC GCA
CCC GCC ATC GGC GAG CAT TGG GCC AAG GGT ACC GCC TGC AAG AGC AGG CCC
CTG AGC CAA GGT GAC TGT CCA CCC CTG GAG TTG AAG AAT ACC GTC CTC GAG
GAC GGC GAC ATG GTG GAC ACC GGC TAC GGC GCA ATG GAT TTC TCC ACC CTG
CAG GAC ACC AAG TGC GAA GTG CCC CTC GAC ATC TGC CAA AGC ATC TGC AAG
TAC CCC GAC TAC CTG CAG ATG AGC GCC GAC CCC TAC GGC GAC TCC ATG TTC
TTC TGT CTG AGA AGG GAA CAA TTG TTC GCC CGC CAC TTC TGG AAC CGC GCC
GGC ACC ATG GGC GAT ACC GTC CCC CAG TCC CTG TAC ATC AAG GGT ACC GGC
ATG AGG GCC AGC CCC GGT TCA TGC GTC TAC AGC CCA AGC CCC TCC GGT AGC
ATC GTC ACA AGC GAT TCC CAA CTC TTC AAC AAG CCC TAC TGG CTG CAC AAA
GCC CAA GGC CAC AAT AAC GGC GTC TGT TGG CAC AAC CAG CTG TTC GTC ACC
GTC GTG GAC ACA ACC AGG TCC ACA AAC CTG ACC ATC TGC GCC AGC ACC CAA
AGC CCC GTG CCC GGC CAG TAC GAC GCC ACA AAG TTC AAA CAA TAC TCA CGC
CAC GTC GAA GAG TAC GAC CTC CAA TTC ATC TTC CAA CTC TGC ACC ATC ACC
CTG ACC GCC GAC GTC ATG TCC TAC ATC CAC TCC ATG AAC TCA TCC ATC CTG
GAA GAC TGG AAT TTC GGT GTC CCA CCA CCC CCC ACC ACC TCC CTC GTC GAC
 
ACC TAC AGG TTC GTG CAG AGC GTC GCC ATC ACA TGC CAG AAA GAC GCC GCC
CCC GCC GAG AAC AAA GAC CCA TAC GAC AAA CTG AAA TTC TGG AAC GTC GAC
CTG AAA GAG AAA TTC AGC CTG GAT CTG GAC CAG TAC CCA TTG GGC AGG AAG
TTC CTC GTC CAG GCG GGT CTC TAA T SEQ ID No.36
18L1ΔCDE131-138/59dE nt 
ATG GCT CTC TGG AGA CCC TCC GAT AAC ACA GTG TAC TTG CCC CCC CCC AGC
GTC GCC CGC GTC GTG AAC ACA GAC GAC TAC GTC ACC AGG ACC TCA ATC TTC
TAC CAC GCC GGT TCA AGC CGC CTG CTG ACC GTC GGC AAC CCC TAC TTC CGC
GTC CCC GCC GGT GGC GGT AAC AAA CAA GAC ATC CCC AAA GTC AGC GCC TAT
CAG TAC CGC GTG TTC CGC GTC CAA CTG CCC GAT CCC AAC AAG TTC GGC CTG
CCC GAC ACC TCC ATC TAC AAC CCC GAG ACC CAG AGG CTG GTC TGG GCA TGC
GCC GGC GTC GAG ATC GGT AGG GGC CAA CCC CTG GGC GTC GGT TTG TCC GGC
CAC CCC TTC TAC AAC AAG CTG GAC GAT ACC GAG TCC GGT CCC CTG TAC AAG
ACC TGC AAG CAG GCC GGT ACC TGC CCC TCC GAC GTC ATC AAC AAG GTC GAA
GGA AAC GTC AGC GAA GAT GTC CGC GAT AAC GTC AGC GTG GAC TAC AAA CAA
ACC CAA CTG TGC ATC CTC GGT TGC GCA CCC GCC ATC GGC GAG CAT TGG GCC
AAG GGT ACC GCC TGC AAG AGC AGG CCC CTG AGC CAA GGT GAC TGT CCA CCC
CTG GAG TTG AAG AAT ACC GTC CTC GAG GAC GGC GAC ATG GTG GAC ACC GGC
TAC GGC GCA ATG GAT TTC TCC ACC CTG CAG GAC ACC AAG TGC GAA GTG CCC
CTC GAC ATC TGC CAA AGC ATC TGC AAG TAC CCC GAC TAC CTG CAG ATG AGC
GCC GAC CCC TAC GGC GAC TCC ATG TTC TTC TGT CTG AGA AGG GAA CAA TTG
TTC GCC CGC CAC TTC TGG AAC CGC GCC GGC ACC ATG GGC GAT ACC GTC CCC
CAG TCC CTG TAC ATC AAG GGT ACC GGC ATG AGG GCC AGC CCC GGT TCA TGC
GTC TAC AGC CCA AGC CCC TCC GGT AGC ATC GTC ACA AGC GAT TCC CAA CTC
TTC AAC AAG CCC TAC TGG CTG CAC AAA GCC CAA GGC CAC AAT AAC GGC GTC
TGT TGG CAC AAC CAG CTG TTC GTC ACC GTC GTG GAC ACA ACC AGG TCC ACA
AAC CTG ACC ATC TGC GCC AGC ACC CAA AGC CCC GTG CCC GGC CAG TAC GAC
GCC ACA AAG TTC AAA CAA TAC TCA CGC CAC GTC GAA GAG TAC GAC CTC CAA
TTC ATC TTC CAA CTC TGC ACC ATC ACC CTG ACC GCC GAC GTC ATG TCC TAC
ATC CAC TCC ATG AAC TCA TCC ATC CTG GAA GAC TGG AAT TTC GGC GTC CCA
CCA CCC CCC ACC ACC TCC CTC GTC GAC ACC TAC AGG TTC GTG CAG AGC GTC
GCC ATC ACA TGC CAG AAA GAC GCC GCC CCC GCC GAG AAC AAA GAC CCA TAC
GAC AAA CTG AAA TTC TGG AAC GTC GAC CTG AAA GAG AAA TTC AGC CTG GAT
CTG GAC CAG TAC CCA TTG GGC AGG AAG TTC CTC GTC CAG GCG GGT CTC TAA
T SEQ ID No.37
18L1DE137-138/59dES-mut1 nt 
ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
 
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT
TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCG GTCCGACGAT TGGCCCTGGC
TCTCGTTCTG CCCCGTCGGC CACGACCAGC AGCGGCCCTG CCGGTAGCGT GAGCGTGGGC
GCTGGCAAAT AAT SEQ ID No.38
18L1DE137-138/59dES-mut2 nt 
ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT
TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCG GTCCGACGAT TGGCCCTCGT
GGCTCTTCTG CCCCGTCGGC CACGACCAGC AGCGGCCCTG CCGGTAGCGT GAGCGTGGGC
GCTGGCAAAT AAT SEQ ID No.39
18L1DE137-138/59dES-mut3 nt 
ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
 
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT
TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCG GTCCGACGAT TGGCCCTGGC
TCTCGTTCTG CCCCGTCGGC CACGACCAGC AGCGGCCCTG CCGGTAGCGT GGGCGTGGAC
GCTGGCAAAT AAT SEQ ID No.40
18L1DE137-138/59dES-mut4 nt 
ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT
TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCG GTCCGACGAT TGGCCCTCGT
 
GGCTCTTCTG CCCCGTCGGC CACGACCAGC AGCGGCCCTG CCGACAGCGT GGGCGTGGAC
GCTGGCAAAT AAT SEQ ID No.41
18L1DE137-138/59dES-mut5 nt 
ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT
TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCA AACCGACGAT TGGCCCTGGC
TCTCGTTCTG CCCCGTCGGC CACGACCAGC AGCGGCCCTG CCGGTAGCGT GAGCGTGGGC
GCTGGCAAAT AAT SEQ ID No.42
18L1DE137-138/59dES-mut6 nt

ATGGCTCTCT GGAGACCCTC CGATAACACA GTGTACTTGC CCCCCCCCAG CGTCGCCCGC
GTCGTGAACA CAGACGACTA CGTCACCAGG ACCTCAATCT TCTACCACGC CGGTTCAAGC
CGCCTGCTGA CCGTCGGCAA CCCCTACTTC CGCGTCCCCG CCGGTGGCGG TAACAAACAA
GACATCCCCA AAGTCAGCGC CTATCAGTAC CGCGTGTTCC GCGTCCAACT GCCCGATCCC
AACAAGTTCG GCCTGCCCGA CACCTCCATC TACAACCCCG AGACCCAGAG GCTGGTCTGG
GCATGCGCCG GCGTCGAGAT CGGTAGGGGC CAACCCCTGG GCGTCGGTTT GTCCGGCCAC
CCCTTCTACA ACAAGCTGGA CGATACCGAG TCCTCCCACG CAGCAACCAG CCTGTACAAG
ACCTGCAAGC AGGCCGGTAC CTGCCCCTCC GACGTCATCA ACGTCAGCGA AGATGTCCGC
GATAACGTCA GCGTGGACTA CAAACAAACC CAACTGTGCA TCCTCGGTTG CGCACCCGCC
ATCGGCGAGC ATTGGGCCAA GGGTACCGCC TGCAAGAGCA GGCCCCTGAG CCAAGGTGAC
TGTCCACCCC TGGAGTTGAA GAATACCGTC CTCGAGGACG GCGACATGGT GGACACCGGC
TACGGCGCAA TGGATTTCTC CACCCTGCAG GACACCAAGT GCGAAGTGCC CCTCGACATC
TGCCAAAGCA TCTGCAAGTA CCCCGACTAC CTGCAGATGA GCGCCGACCC CTACGGCGAC
TCCATGTTCT TCTGTCTGAG AAGGGAACAA TTGTTCGCCC GCCACTTCTG GAACCGCGCC
GGCACCATGG GCGATACCGT CCCCCAGTCC CTGTACATCA AGGGTACCGG CATGAGGGCC
AGCCCCGGTT CATGCGTCTA CAGCCCAAGC CCCTCCGGTA GCATCGTCAC AAGCGATTCC
CAACTCTTCA ACAAGCCCTA CTGGCTGCAC AAAGCCCAAG GCCACAATAA CGGCGTCTGT

TGGCACAACC AGCTGTTCGT CACCGTCGTG GACACAACCA GGTCCACAAA CCTGACCATC
TGCGCCAGCA CCCAAAGCCC CGTGCCCGGC CAGTACGACG CCACAAAGTT CAAACAATAC
TCACGCCACG TCGAAGAGTA CGACCTCCAA TTCATCTTCC AACTCTGCAC CATCACCCTG
ACCGCCGACG TCATGTCCTA CATCCACTCC ATGAACTCAT CCATCCTGGA AGACTGGAAT
TTCGGCGTCC CACCACCCCC CACCACCTCC CTCGTCGACA CCTACAGGTT CGTGCAGAGC
GTCGCCATCA CATGCCAGAA AGACGCCGCC CCCGCCGAGA ACAAAGACCC ATACGACAAA
CTGAAATTCT GGAACGTCGA CCTGAAAGAG AAATTCAGCC TGGATCTGGA CCAGTACCCA
TTGGGCAGGA AGTTCCTCGT CCAGGCGGGT CTCCGTGGCA AACCGACGAT TGGCCCTCGT
GGCTCTTCTG CCCCGTCGGC CACGACCAGC AGCGGCCCTG CCGGTAGCGT GAGCGTGGGC
GCTGGCAAAT AAT SEQ ID No.43

### Example 3: Construction of recombinant bacmids and recombinant baculoviruses of the chimeric L1 protein genes

Recombinant expression vectors comprising the chimeric L1 genes, namely pFastBac1-18L1ΔCDE137-138/59dES, pFastBac1-18L1ΔCDE137-138/59dE, pFastBac1-18L1ΔCh4432-433/59dE, pFastBac1-18L1ΔCh4434-435/59dE, pFastBac1-18L1ΔCDE134-135/59dES, pFastBac1-18L1ΔCDE134-135/59dE, pFastBac1-18L1ΔCDE131-138/59dE, pFastBac1-18L1ΔCDE121-124/59dE, pFastBac1-18L1ΔCh4431-433/59dE, pFastBac1-18L1ΔCh4432-435/59dE, pFastBac1-18L1DE137-138/59dES-mut1, pFastBac1-18L1DE137-138/59dES-mut2, pFastBac1-18L1DE137-138/59dES-mut3, pFastBac1-18L1DE137-138/59dES-mut4, pFastBac1-18L1DE137-138/59dES-mut5, pFastBac1-18L1DE137-138/59dES-mut6, were used to transform E. *coli* DH10Bac competent cells, which were screened to obtain recombinant Bacmids. Then the recombinant Bacmids were used to transfect Sf9 insect cells so as to amplify recombinant baculoviruses within the Sf9 cells. Methods of screening of recombinant Bacmids and amplification of recombinant baculoviruses were all well known, for example, the patent CN 101148661 B.

### Example 4: Expression of chimeric L1 protein genes in Sf9 cells

Sf9 cells were inoculated with the recombinant baculoviruses of the 16 chimeric L1 genes to express the chimeric L1 proteins. After incubation at 27°C for about 88 h, the fermentation broth was collected and centrifuged at 3,000 rpm for 15 min. The supernatant was discarded, and the cells were washed with PBS for use in expression identification and purification. Methods of infection and expression were publicly available, for example, the patent CN 101148661 B.

### Example 5: Expression of chimeric L1 protein genes in E. coli

Recombinant expression vectors comprising the chimeric L1 genes, namely pET22b-18L1DE137-138/59dES, pET22b-18L1DE137-138/59dE, pET22b-18L1h4432-433/59dE, pET22b-18L1h4434-435/59dE, pET22b-18L1DE134-135/59dES, pET22b-18L1DE134-135/59dE, pET22b-18L1DE131-138/59dE, pET22b-18L1DE121-124/59dE, pET22b-18L1h4431-433/59dE, pET22b-18L1h4432-435/59dE, pET22b-18L1DE137-138/59dES-mut1, pET22b-18L1DE137-138/59dES-mut2, pET22b-18L1DE137-138/59dES-mut3, pET22b-18L1DE137-138/59dES-mut4, pET22b-18L1DE137-138/59dES-mut5, pET22b-18L1DE137-138/59dES-mut6, were used to transform *E. coli* BL21 (DE3).

Single clones were picked and inoculated into 3 ml of LB medium containing ampicillin and incubated at 37°C overnight. The bacterial fluid cultured overnight was added to LB medium at a ratio of 1:100 and incubated at 37°C for about 3 h. When the OD600 reached between 0.8-1.0, IPTG was added to a final concentration of 0.5 µM, and the bacterial fluid was incubated at 16°C for about 12 h and collected.

### Example 6: Identification of the expression of the chimeric L1 proteins

1×106 cells expressing the different chimeric L1 proteins described in Examples 4 and 5 respectively were collected and resuspended in 200 µl of PBS solution. 50 µl of 6× loading buffer was added and the samples were denatured at 75°C for 8 minutes. 10 µl of sample was used for SDS-PAGE electrophoresis and Western blot identification, respectively. The results were as shown in Figure 1A to Figure 1B. All 26 chimeric L1 proteins could be expressed at high levels in insect cells or prokaryotic expression systems, among which 18L1DE137-138/59dES, 18L1DE137-138/59dE, 18L1h4432-433/59dE, 18L1h4434-435/59dE, 18L1DE134-135/59dES, 18L1DE134-135/59dE, 18L1DE131-138/59dE, 18L1DE121-124/59dE, 18L1h4431-433/59dE, 18L1h4432-435/59dE, 18L1DE137-138/59dES-mut1, 18L1DE137-138/59dES-mut2, 18L1DE137-138/59dES-mut3, 18L1DE137-138/59dES-mut4, 18L1DE137-138/59dES-mut5, 18L1DE137-138/59dES-mut6 were about 59 kDa in size, while the rest 10 proteins were about 55 kDa in size. Methods of SDS-PAGE electrophoresis and Western blot identification were publicly available, for example, the patent CN 101148661 B.

### Example 7: Comparison of expression amount of chimeric L1 proteins in insect cells

1×106 cells expressing the 18L1 backbone protein with 32-amino acid truncation at C-terminus, as well as cells expressing the chimeric L1 proteins with the 18L1 with 32-amino acid truncation at C-terminus as backbone, or with the 6 types of 18L1 mutants as backbone, respectively, were collected and resuspended in 200 µl of PBS solution. The cells were disrupted by ultrasonic disruption (Ningbo Scientz Ultrasonic Cell Disruptor, 2# probe, 100 W, ultrasound 5 s, interval 7 s, total time 3 min) and centrifuged at a high speed of 12,000 rpm for 10 minutes. The lysed supernatant was collected and the L1 content in the supernatant was detected by sandwich ELISA, which was well known, for example, the patent CN104513826A.

Microtiter plates were coated with HPV18 L1 monoclonal antibodies prepared by the inventor at 80 ng/well by overnight incubation at 4°C. The plate was blocked with 5% BSA-PBST at room temperature for 2 h and washed 3 times with PBST. The lysed supernatant was subjected to 2-fold serial dilution with PBS. The HPV18L1 VLP standard was also subjected to serial dilution from a concentration of 2 µg/ml to 0.0625 µg/ml. The diluted samples were added to the plate respectively at 100 µl per well and incubated at 37°C for 1 h. The plate was washed 3 times with PBST, and 1:3000 diluted HPV18L1 rabbit polyclonal antibody was added at 100 µl per well and incubated at 37°C for 1 h. The plate was washed 3 times with PBST, and 1:3000 diluted HRP-labeled goat anti-mouse IgG (1:3000 dilution, ZSGB-Bio Corporation) was added and incubated at 37°C for 45 minutes. The plate was washed 5 times with PBST, and 100 µl of OPD substrate (Sigma) was added to each well for chromogenic reaction at 37°C for 5 minutes. The reaction was stopped with 50 µl of 2 M sulfuric acid, and the absorbance at 490 nm was determined. The concentrations of HPV18L1 protein and 18L1 chimeric proteins in the lysed supernatant were calculated according to the standard curve.

The results were as shown in Table 3. The expression amounts of 18L1ΔCDE134-135/59dES, 18L1ΔCDE137-138/59dES, 18L1ΔCh4431-433/59dE and 18L1ΔCh4432-435/59dE of the present invention were very high, comparable to that of the HPV18L1 backbone. In addition, the expression amounts of the chimeric proteins with the 18L1 mutants with amino acid substitutions at C-terminus as backbone, namely 18L1DE137-138/59dES-mut1, 18L1DE137-138/59dES-mut4, 18L1DE137-138/59dES-mut5, were all higher than that of the HPV18L1 backbone and the corresponding C-terminal truncated chimeric protein.

**Table 3: Analysis of expression amounts of chimeric L1 proteins**

| Protein name | Expression amount (mg/L) | | | |
|---|---|---|---|---|
| | Batch 1 | Batch 2 | Batch 3 | Average |
| HPV18L1 | 55 | 52 | 59 | 55.3 |
| 18L1ΔCDE137-138/59dES | 59 | 51 | 54 | 54.7 |
| 18L1ΔCDE137-138/59dE | 43 | 41 | 46 | 43.3 |
| 18L1ΔCh4432-433/59dE | 29 | 29 | 30 | 29.3 |
| 18L1ΔCh4434-435/59dE | 46 | 44 | 44 | 44.7 |
| 18L1ΔCDE134-135/59dES | 56 | 58 | 53 | 55.7 |
| 18L1ΔCDE134-135/59dE | 43 | 43 | 41 | 42.3 |
| 18L1ΔCDE131-138/59dE | 41 | 39 | 40 | 40 |
| 18L1ΔCDE121-124/59dE | 45 | 42 | 39 | 42 |
| 18L1ΔCh4431-433/59dE | 52 | 59 | 57 | 56 |
| 18L1ΔCh4432-435/59dE | 54 | 55 | 51 | 53.3 |
| 18L1DE137-138/59dES-mut1 | 75 | 72 | 77 | 74.7 |
| 18L1DE137-138/59dES-mut2 | 42 | 39 | 35 | 38.7 |
| 18L1DE137-138/59dES-mut3 | 32 | 28 | 36 | 32 |
| 18L1DE137-138/59dES-mut4 | 78 | 80 | 76 | 78 |
| 18L1DE137-138/59dES-mut5 | 81 | 79 | 83 | 81 |
| 18L1DE137-138/59dES-mut6 | 50 | 55 | 52 | 52.3 |

### Example 8: Purification and dynamic light scattering particle size analysis of the chimeric L1 proteins

An appropriate amount of cell fermentation broth of chimeric L1 was collected and the cells were resuspended with 10 ml of PBS. PMSF was added to a final concentration of 1 mg/ml. The cells were ultrasonically disrupted (Ningbo Scientz Ultrasonic Cell Disruptor, 6# probe, 200 W, ultrasound 5 s, interval 7 s, total time 10 min) and the disrupted supernatant was collected for purification. The purification steps were carried out at room temperature. 4% β-mercaptoethanol (w/w) was added to the lysate to disaggregate VLP. Then the samples were filtered with 0.22 µm filters, followed by sequential purification with DMAE anion exchange chromatography or CM cation exchange chromatography (20 mM Tris, 180 mM NaCl, 4% β-ME, elution at pH 7.9), TMAE anion exchange chromatography or Q cation exchange chromatography (20 mM Tris, 180 mM NaCl, 4% β-ME, elution at pH 7.9) and hydroxyapatite chromatography (100 mM NaH2PO4, 30 mM NaCl, 4% β-ME, elution at pH 6.0). The purified product was concentrated and buffer (20 mM NaH2PO4, 500 mM NaCl, pH 6.0) exchange was performed using Planova ultrafiltration system to prompt VLP assembly. The above purification methods were all publicly available, for example, the patents CN 101293918 B, CN 1976718 A, etc.

Severe aggregation was found for 18L1h4431-433/59dE, 18L1h4432-435/59dE, 18L1ΔCh4431-433/59dE and 18L1ΔCh4432-435/59dE during the assembly of pure chimeric proteins, while aggregation was not observed for other chimeric proteins after assembly. The assembled chimeric protein solutions were subjected to DLS particle size analysis (Zetasizer Nano ZS 90 Dynamic Light Scattering Analyzer, Malvern), and the results were as shown in Table 4. Among them, the DLS analysis plots of 18L1ΔCDE134-135/59dE, 18L1ΔCDE134-135/59dES, 18L1ΔCDE137-138/59dE, 18L1ΔCDE137-138/59dES were as shown in Figure 2A to Figure 2D.

**Table 4: DLS analysis of chimeric L1 proteins**

| Protein name | Hydraulic diameter (nm) | PDI |
|---|---|---|
| 18L1DE137-138/59dES | 115.2 | 0.182 |
| 18L1DE137-138/59dE | 119.1 | 0.172 |
| 18L1h4432-433/59dE | 125.4 | 0.163 |
| 18L1h4434-435/59dE | 114.2 | 0.177 |
| 18L1DE134-135/59dES | 104.3 | 0.193 |
| 18L1DE134-135/59dE | 106.8 | 0.169 |
| 18L1DE131-138/59dE | 113.3 | 0.192 |
| 18L1DE121-124/59dE | 114.7 | 0.133 |
| 18L1ΔCDE137-138/59dES | 122.9 | 0.192 |
| 18L1ΔCDE137-138/59dE | 121.4 | 0.188 |
| 18L1ΔCh4432-433/59dE | 107.5 | 0.179 |
| 18L1ΔCn4434-435/59dE | 105.2 | 0.196 |
| 18L1ΔCDE134-135/59dES | 112.4 | 0.133 |
| 18L1ΔCDE134-135/59dE | 110.5 | 0.167 |
| 18L1ΔCDE131-138/59dE | 109.2 | 0.184 |
| 18L1ΔCDE121-124/59dE | 121.7 | 0.165 |
| 18L1DE137-138/59dES-mut1 | 120.5 | 0.178 |
| 18L1DE137-138/59dES-mut2 | 118.4 | 0.165 |
| 18L1DE137-138/59dES-mut3 | 115.7 | 0.182 |
| 18L1DE137-138/59dES-mut4 | 119.2 | 0.133 |
| 18L1DE137-138/59dES-mut5 | 122.3 | 0.142 |
| 18L1DE137-138/59dES-mut6 | 125.6 | 0.191 |

### Example 9: Transmission electron microscopy observation of chimeric VLPs

The chimeric proteins were purified separately according to the chromatographic purification method described in Example 8. The assembled chimeric proteins were prepared on copper mesh, stained with 1% uranium acetate, fully dried and then observed using JEM-1400 electron microscope (Olympus). The results showed that the chimeric proteins expressed by both E. *coli* and insect cells could be assembled into cVLPs with a diameter of about 50 nm. Among them, the electron microscopy images of 18L1ΔCDE134-135/59dE, 18L1ΔCDE134-135/59dES, 18L1ΔCDE137-138/59dE, 18L1ΔCDE137-138/59dES cVLPs were as shown in Figure 3A to Figure 3D. Methods of copper mesh preparation and electron microscopy observation were all publicly available, for example, the patent CN 101148661 B.

### Example 10: Immunization of mice with chimeric VLPs and determination of neutralizing antibody titers

4-6 weeks old BALB/c mice were randomly divided into groups, 5 mice per group, and 10 µg cVLP in combination with 50 µg Al(OH)3 and 5 µg MPL adjuvant were used to immunize mice by subcutaneous injection at Weeks 0, 4, 7, 10 for a total of 4 times. Tail vein blood was collected 2 weeks after the 4th immunization and serum was isolated.

24 types of HPV pseudoviruses were used to detect the neutralizing antibody titers of the immune serum. The results showed that after immunizing mice with various cVLPs produced *by E. coli* and insect cell expression systems, the levels and neutralization range of the induced cross-neutralizing antibodies were different with each other. Among them, as shown in Table 5, the antiserum of 18L1ΔCDE134-135/59dES and 18L1ΔCDE137-138/59dES cVLPs expressed by insect cells could neutralize at least 23 types of pseudoviruses, and the immune serum of 18L1ΔCDE134-135/59dE and 18L1ΔCDE137-138/59dE cVLPs could neutralize at least 19 types of pseudoviruses. It was worth mentioning that the antiserum of 18L1ΔCDE134-135/59dES and 18L1ΔCDE137-138/59dES cVLP could neutralize all 6 α7-HPVs used for detection, in particular for 18L1ΔCDE137-138/59dES cVLP, the antibody titers to cross-neutralize α7-HPVs were all above 250, and it was the cVLP with the strongest cross-neutralization ability against α7-HPVs reported so far.

In addition, after immunizing mice with the above strategy, the cVLPs constructed with 18L1 mutants with 32-amino acid truncation at C-terminus in the present invention could also induce high levels of neutralizing antibodies. Among them, the level of each type of neutralizing antibody induced by 18L1DE137-138/59dES-mut4 was comparable to those induced by 18L1ΔCDE137-138/59dES. It was worth noticing that the HPV39 and HPV59 neutralizing antibody titers of 18L1DE137-138/59dES-mut4 immune serum were both greater than 103 (as shown in Table 5 and Figure 4).

Methods of pseudovirus preparation and pseudoviral neutralization experiments were all publicly available, for example, the patent CN 104418942A.

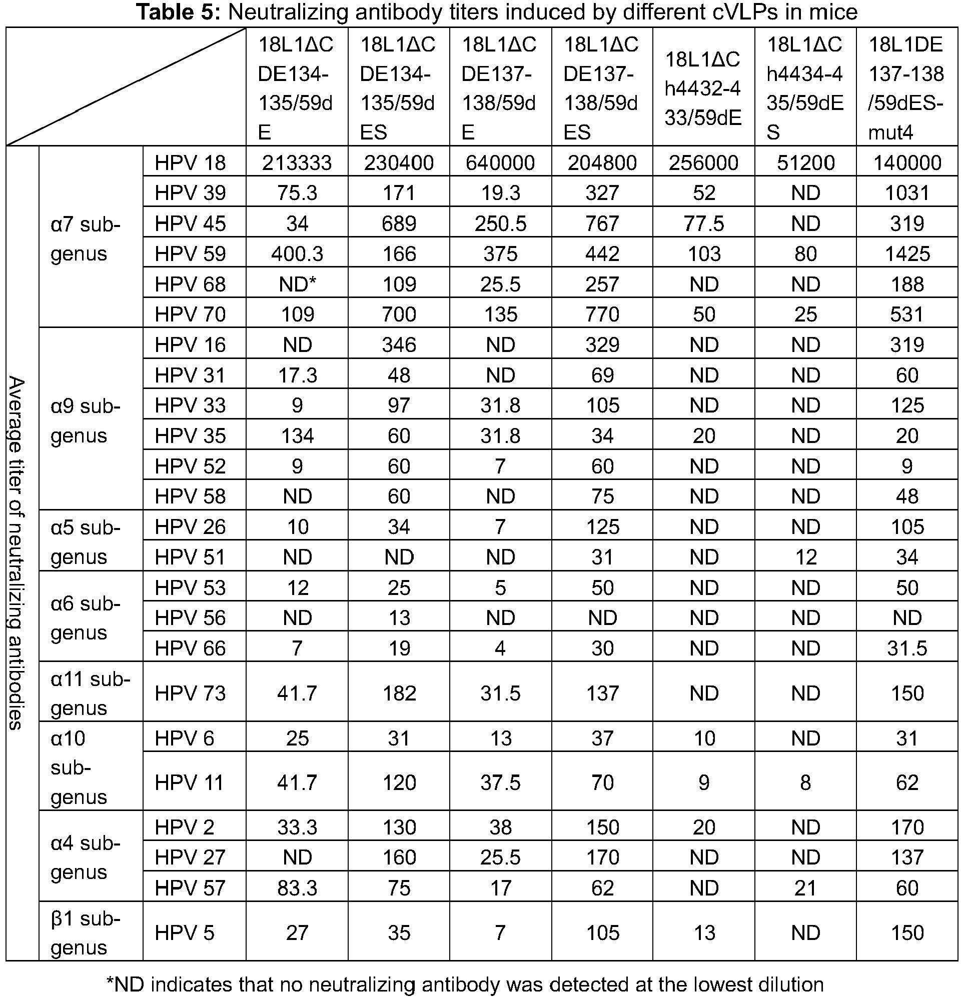

## Claims

1. A human papillomavirus chimeric protein comprising or consisting of a HPV type 18 L1 protein or a mutant of the HPV type 18 L1 protein, and a polypeptide from a HPV type 59 L2 protein inserted into the surface region of the HPV type 18 L1 protein or the mutant of the HPV type 18 L1 protein, wherein the amino acid sequence of the HPV type 18 L1 protein is as shown in SEQ ID No. 1, and the amino acid sequence of the HPV type 59 L2 protein is as shown in SEQ ID No. 2, preferably, the mutant of the HPV type 18 L1 protein is selected from any one of the group consisting of:
a mutant with a 32-amino acid truncation at C-terminus of the amino acid sequence as shown in SEQ ID No. 1;
a mutant with amino acid substitutions of amino acids 477, 478, 484, 496, 499, 504, 506 to glycine (G) and amino acids 485, 500, 502 to serine (S) in the amino acid sequence as shown in SEQ ID No. 1;
a mutant with amino acid substitutions of amino acids 477, 478, 485, 496, 499, 504, 506 to glycine (G) and amino acids 486, 500, 502 to serine (S) in the amino acid sequence as shown in SEQ ID No. 1;
a mutant with amino acid substitutions of amino acids 477, 478, 484, 496, 499, 502, 506 to glycine (G), amino acids 485, 500 to serine (S) and amino acid 504 to aspartate (D) in the amino acid sequence as shown in SEQ ID No. 1;
a mutant with amino acid substitutions of amino acids 477, 478, 485, 496, 502, 506 to glycine (G), amino acids 486, 500 to serine (S) and amino acids 499, 504 to aspartate (D) in the amino acid sequence as shown in SEQ ID No. 1;
a mutant with amino acid substitutions of amino acids 477, 484, 496, 499, 504, 506 to glycine (G) and amino acids 485, 500, 502 to serine (S) in the amino acid sequence as shown in SEQ ID No. 1; and
a mutant with amino acid substitutions of amino acids 477, 485, 496, 499, 504, 506 to glycine (G) and amino acids 486, 500, 502 to serine (S) in the amino acid sequence as shown in SEQ ID No. 1;
more preferably, the polypeptide from HPV type 59 L2 protein is selected from any of the polypeptides as shown in SEQ ID No. 3, SEQ ID No. 4, SEQ ID No. 5 and SEQ ID No. 6;
further preferably, the polypeptide from the HPV type 59 L2 protein is inserted into the surface region of the HPV type 18 L1 protein or the mutant of the HPV type 18 L1 protein, the surface region is a DE loop and/or h4 region, preferably, the polypeptide from the HPV type 59 L2 protein is inserted between amino acids 137 and 138, between amino acids 432 and 433, or between amino acids 434 and 435 of the HPV type 18 L1 protein or the mutant of the HPV type 18 L1 protein by direct insertion, alternatively, the polypeptide from the HPV type 59 L2 protein is inserted into the region of amino acids 121 to 124, or the region of amino acids 131 to 138, or the region of amino acids 431 to 433, or the region of amino acids 432 to 435 of the HPV type 18 L1 protein or the mutant of the HPV type 18 L1 protein by non-isometric substitution;
more preferably, wherein the polypeptide from the HPV type 59 L2 protein comprises a linker of 1 to 3 amino acid residues at its N- and/or C-terminus, the linker comprises one or more amino acids selected from the group consisting of glycine, serine, alanine and proline, preferably, the linker at N-terminus consists of glycine-proline, and the linker at C-terminus consists of proline.

2. The human papillomavirus chimeric protein according to claim 1, wherein the amino acid sequence of the human papillomavirus chimeric protein is as shown in any one of SEQ ID Nos. 7-32.

3. A polynucleotide encoding the human papillomavirus chimeric protein according to claim 1 or 2, preferably, the sequence of the polynucleotide is whole-gene optimized using *E. coli* codons or whole-gene optimized using insect cell codons, more preferably, the sequence of the polynucleotide is as shown in any one of SEQ ID No. 33, SEQ ID No. 34, SEQ ID No. 35, SEQ ID No. 36, SEQ ID No. 37, SEQ ID No. 38, SEQ ID Any of No.39, SEQ ID No. 40, SEQ ID No. 41, SEQ ID No. 42 and SEQ ID No. 43.

4. A vector comprising the polynucleotide according to claim 3.

5. A cell comprising the vector according to claim 4.

6. A polymer which is a chimeric pentamer or chimeric virus-like particle comprising the human papillomavirus chimeric protein according to claim 1 or 2, or formed by the human papillomavirus chimeric protein according to claim 1 or 2.

7. Use of the human papillomavirus chimeric protein according to claim 1 or 2 or the polymer according to claim 6 in the preparation of a vaccine for the prevention of papillomavirus infection and/or papillomavirus infection-induced diseases, preferably, the papillomavirus infection-induced diseases are selected from the group consisting of cervical cancer, vaginal cancer, vulval cancer, penile cancer, perianal cancer, oropharyngeal cancer, tonsil cancer, and oral cancer;
preferably, the papillomavirus infection is an infection selected from one or more of the following papillomavirus types: HPV16, HPV18, HPV26, HPV31, HPV33, HPV35, HPV39, HPV45, HPV51, HPV52, HPV53, HPV56, HPV58, HPV59, HPV66, HPV68, HPV70, HPV73; HPV6, HPV11, HPV2, HPV5, HPV27 and HPV57.

8. A vaccine for the prevention of papillomavirus infection and/or papillomavirus infection-induced diseases, comprising the human papillomavirus chimeric protein according to claim 1 or 2 or the polymer according to claim 6, an adjuvant, as well as an excipient or carrier for vaccines.

9. The vaccine for the prevention of papillomavirus infection and/or papillomavirus infection-induced diseases according to claim 8, further comprising at least one virus-like particle or chimeric virus-like particle of mucosa-tropic and/or skin-tropic HPVs.

10. The vaccine for the prevention of papillomavirus infection and/or papillomavirus infection-induced diseases according to claim 8 or 9, wherein the adjuvant is for use in human.
